# EUROPEAN PATENT APPLICATION

(11) **EP 2 045 263 A1**
(43) Date of publication of application: **08.04.2009**
(21) Application number: 07121688.1
(22) Date of filing: 27.11.2007
(51) Int. Cl.: C07K 14/435

(54) **Identification and molecular characterisation of salivary metalloproteases expressed in the tick salivary glands**

(30) Priority: 02.10.2007 EP 07117796
(71) Applicant: UNIVERSITE LIBRE DE BRUXELLES, 1050 Brussels (BE)
(72) Inventor: Godfroid, Edmond, B-1120, BRUXELLES (BE); Decrem, Yves, B-1380, OHAIN (BE); Vanhamme, Luc, B-1490, COURT-SAINT-ETIENNE (BE)
(74) Representative: pronovem

(57) **Abstract**

An isolated and purified polypeptide obtained from tick salivary gland and presenting more than 75% sequence identity with at least a sequence selected from the group consisting of SEQ.ID.NO.8 or SEQ.ID.NO.9.

## Description

### Field of the invention

The present invention relates to the molecular characterisation of DNA sequences which encode metalloproteases expressed in the salivary glands of ticks, more particularly the *Ixodes ricinus* tick. The invention relates also to newly identified polypeptides, polynucleotides, encoding them and to the use of such polypeptides and polynucleotides.

### Background of the invention

Ticks are blood feeding acarines comprised of three families. They infest a large variety of vertebrates ranging from amphibians to mammals, including domestic animals and humans. As they threaten the health of their hosts through anemia subsequent to blood puncture, poisoning by saliva or transmission of pathogens, they are of prime medical and veterinary interest. For example, *Ixodes ricinus* is the vector of Babesiosis, Erlichiosis, Tick Borne Encephalitis and of Lyme disease in Europe.

In order to obtain their blood meal, ticks anchor onto their hosts, uptake blood and then fall off upon repletion. Contrarily, to many other blood feeding arthropods, ticks of the Ixodidae family including *Ixodes ricinus* remain attached to their hosts for long time periods, sometimes several weeks. Therefore, to complete the whole process, they have to counteract various barriers and defence mechanisms of the host. They therefore have to disrupt tissues and fight against processes such as haemostasis, immune reaction or pain.

While part of this action is performed at the physical level by the rostrum, most of it is ensured by the chemical effects of the salivary components injected during the entire length of the meal. Thus, whole salivary gland extracts or saliva have both been reported to inhibit different host defences. Many of the components of ticks' saliva that are able to inhibit pain sensation, counteract the immune system, or interfere with various pathways of haemostasis, have been characterized. Some examples are: Salp 15 or Iris (Anguita et al., 2002, Leboulle et al., 2002) that suppress the immune reaction. A metallo-dipeptidyl-carboxypeptidase (Ribeiro and Mather, 1998) and proteins of the RaHBP family (Paesen et al., 1999), which are able to bind histamine, thereby decreasing inflammation and inhibiting pain and itching respectively. Haemaphysalin (Kato et al., 2005) and Ixolaris or TCI (Francischetti et al., 2002, Arolas et al., 2005) were found to interfere with haemostasis, as an anticoagulant and as fibrinolysis activating factors respectively. In addition, an aspartic protease able to hydrolyze haemoglobin has been characterized in *Haemaphysalis longicornis* (Boldbaatar et al., 2006). Proteases or protease inhibitors are putative weapons of the parasite.

To date, metalloproteases have been found to be involved in (among other roles) tissue remodeling or disruption through digestion of structural components (Francischetti et al., 2003; Francischetti et al., 2005). Inoculation of metalloproteases found in the saliva of various biting animals also participates in disrupting the balance of haemostasis. For example, the toxicity of snake venom is partly due to several metalloproteases that interfere with haemostasis, disrupt basal laminae or extracellular matrices and trigger haemorrhages (Ramos and Selistre-de-Araujo, 2006). Metalloproteases could perform similar functions in the saliva of blood feeding arthropods and related metalloprotease was also found to be essential for completion of the blood meal.

### Summary of the invention

The present invention is related to an isolated and purified (poly)peptide (or protein) obtained from tick salivary gland and presenting more than 50%, 55%, 60%, 65%, 70%, 75% sequence identity with at least an amino acid sequence selected from the group consisting of SEQ.ID.NO.8 (Metis 3) or SEQ.ID.NO.9 (Metis 4).

Preferably, the (poly)peptide of the invention presents at least 80% or 85% sequence identity with at least one of these sequences, more preferably at least 90% sequence identity, more preferably at least 95%, 96%, 97% sequence identity, more preferably between 98 to 99% , more preferably at least 99% sequence identity with SEQ.ID.NO.8 or SEQ.ID.NO.9.

The present invention is also related to a (poly)peptide sequence comprising or consisting of the sequence SEQ.ID.NO.8 (Metis 3) or SEQ.ID.NO.9 (Metis 4),a variant thereof, or a biologically active fragment or portion thereof (which means a sequence of this (poly)peptide which presents the same biological activity than the complete sequence, or an antigenic or immunogenic fragment or portion thereof).

The present invention is also related to a (poly)nucleotide (preferably the nucleotide sequence SEQ.ID.NO.3 or SEQ.ID.NO.4), encoding the polypeptide of the present invention a variant or a biologically active (antigenic or immunogenic) fragment or portion thereof.

This polypeptide may be modified by or linked to at least one substitution group, preferably selected from the group consisting of amide, acetyl, phosphoryl, and/or glycosyl groups. Moreover, this polypeptide may take the form of a "mature" protein. It may also be part of a larger protein or part of a fusion protein.

Preferably, the polypeptide of the present invention further includes at least one additional amino acid sequence which contains secretory or leader sequences, pro-sequences, sequences which help in purification such as multiple histidine residues, or additional sequences for stability during recombination protection.

Another object of the present invention concerns a variant of the polynucleotide or the polypeptide of the present invention, a precise definition of this term being given hereafter. Preferably, said variant varies from the referent by conservative amino acid substitutions. Preferably, at least one residue is substituted in said variant with another residue of similar characteristics. Advantageously, the substitutions in said variant are among Ala, Val, Leu and Ile; among Ser and Thr, among the acidic residues Asp and Glu; among Asn and Gln; among the basic residues Lys and Arg; or among aromatic residues Phe and Tyr.

Preferably, in the variant of the present invention, several amino acids are substituted, deleted or added in any combination. Preferably, 5 to 10, more preferably 1 to 5, more preferably 1 to 2 amino acids are substituted, deleted or added in any combination, in said variant.

Said variant may be a naturally occurring allelic variant of an *Ixodes ricinus* salivary gland polypeptide present in *Ixodes ricinus* salivary glands.

The present invention is also related to a vector comprising at least one element selected from the group consisting of the polynucleotide, the polypeptide, (the variant), according to the present invention and active fragments thereof.

Another object of the present invention concerns a cell transfected by or comprising the recombinant vector according to the invention.

The present invention further discloses an inhibitor (anti-sense sequence, ribozyme, antibody, hypervariable portion thereof, nanobody, etc.) raised against the polynucleotide, the polypeptide, or the variant, according to the present invention.

The present invention is also related to an hybridoma cell line expressing said antibody.

Another object of the present invention concerns a pharmaceutical composition comprising adequate pharmaceutical carrier and an element selected from the group consisting of said polynucleotide, polypeptide, variant, vector, cell, inhibitor or a mixture thereof. Preferably, said pharmaceutical composition presents immunomodulatory properties.

Another object of the invention is an immunological composition or vaccine for inducing an immunological response in a mammalian host to a tick salivary gland polypeptide which comprises at least one element of the group consisting of
a) a tick salivary gland polynucleotide according to the invention;
b) a tick salivary gland polypeptide according to the invention;
c) possibly the variant according to the invention;
d) epitope-bearing fragments, analogs, outer-membrane vesicles or cells (attenuated or otherwise) of components a) or b) or c);
e) and possibly an adequate pharmaceutical carrier or diluent.

The present invention is also related to the use of the immunological composition, the vaccine, the vector, according to the invention to manufacture a medicament to produce antibody and/or T-cell immune response to protect a mammal from bacteria and viruses and related species and in the treatment or the prevention of Lyme diseases or tick encephalitis virus diseases.

The present invention is also related to a method for treating or preventing a disease affecting a mammal, said method comprising the step of administrating to said mammal a sufficient amount of the pharmaceutical composition according to the invention or the immunological composition or vaccine according to the invention, to prevent or cure the transmission of pathogenous agents by tick, especially by *Ixodes ricinus,* or the symptoms of diseases induced by tick or pathogenous agents transmitted by tick.

The present invention is also related to a diagnostic kit for detecting a disease or susceptibility to a disease induced or transmitted by tick, especially *Ixodes ricinus,* which comprises:
a) the tick salivary gland polynucleotide according to the invention or an active fragment thereof;
b) the nucleotie sequence complementary to that a);
c) the tick salivary gland polypeptide according to the invention or an active fragment thereof;
d) possibly the variant according to the invention or an active fragment thereof;
e) the inhibitor (preferably the antibody) according to the invention;
f) a phage displaying the inhibitor (preferably the antibody) according to the invention,
whereby a), b), c), d), e) f) may comprise a substantial component.

The *I. ricinus* salivary gland polypeptides of the present invention may be in the form of the "mature" protein or may be a part of a larger protein such as a fusion protein. It may be advantageous to include an additional amino acid sequence which contains secretory or leader sequences, pro-sequences, sequences which help in purification such as multiple histidine residues, or an additional sequence for stability during recombinant production.

When the polynucleotides of the invention are used for the production of an *I. ricinus* salivary gland recombinant polypeptide, the polynucleotide may include the coding sequence for the mature polypeptide or a fragment thereof, by itself; the coding sequence for the mature polypeptide or fragment in reading frame with other coding sequences, such as those encoding a leader or secretory sequence, a pre-, or pro-or preproprotein sequence, or other fusion peptide portions. For example, a marker sequence, which facilitates purification of the fused polypeptide can be encoded. Preferably, the marker sequence is a hexa-histidine peptide, as provided in the pQE vector (Qiagen, Inc.) or is an HA tag, or is glutathione-s-transferase. The polynucleotide may also contain non-coding 5' and 3' sequences, such as transcribed, non-translated sequences, splicing and poly-adenylation signals, ribosome binding sites and sequences that stabilize mRNA.

The present invention further relates to inhibitors being polynucleotides (antisense sequences, ribozymes,...) that hybridise preferably stringent conditions to the herein above-described sequences. As herein used, the term "stringent conditions" means hybridisation will occur only if there is at least 80%, and preferably at least 90%, and more preferably at least 95%, yet even more preferably 97-99% identity between the sequences.

The anti-*I*. *ricinus* salivary gland polypeptide inhibitors, which may be polyclonal antibodies, are prepared by any suitable methods known by the skilled person. Polyclonal antibodies can be raised in a mammal for example, by one or more injections of an immunizing agent and, if desired, an adjuvant. Typically, the immunizing agent and/or adjuvant will be injected in the mammal by multiple subcutaneous or intraperitoneal injections. The immunizing agent may include the *I*. *ricinus* salivary gland polypeptides of the invention or a fusion protein thereof. It may be useful to conjugate the immunizing agent to a protein known to be immunogenic in the mammal being immunized. Examples of such immunogenic proteins include but are not limited to keyhole limpet hemocyanin, serum albumin, bovine thyroglobulin, and soybean trypsin inhibitor. Examples of adjuvants which may be employed include Freund's complete adjuvant and MPL TDM adjuvant The immunization protocol may be selected by one skilled in the art without undueexperimentation.

The anti-*I*. *ricinus* salivary gland polypeptide inhibitors may, alternatively, be monoclonal antibodies. Monoclonal antibodies may be prepared using any suitable methods, for example, hybridoma methods, such as those described by Kohler et al. (1975). In a hybridoma method, a mouse, hamster, or other appropriate host animal, is typically immunized with an immunizing agent to elicit lymphocytes that produce or are capable of producing antibodies that will specifically bind to the immunizing agent. Alternatively, the lymphocytes may be immunized in vitro.

The pharmaceutical composition and the immunological composition or vaccine according to the invention, can be prepared by any suitable method.

Furthermore, a diagnostic kit for a disease or susceptibility to a disease which comprises:
a) an *I. ricinus* salivary gland polynucleotide, preferably the nucleotide sequence of one of the gene sequences defined in the sequence listing, or a fragment thereof;
b) a nucleotide sequence complementary to that of a);
c) an *I. ricinus* salivary gland polypeptide, preferably the polypeptide encoded by one of the gene sequences defined in the sequence listing, or a fragment thereof;
d) an inhibitor (antibody) to an *I. ricinus* salivary gland polypeptide or polynucleotide, preferably to the polypeptide encoded by one of the gene sequences defined in the sequence listing; or
e) a phage displaying an inhibitor (antibody) to an *I. ricinus* salivary gland polypeptide, preferably to the polypeptide encoded by one of the cDNAs sequences defined in the sequence listing; can be prepared by any suitable method. It will be appreciated that in any such kit, a), b), c), d) or e) may comprise a substantial component.

The immunological composition, or vaccine, or the vector, according the invention, can be used to manufacture a medicament to produce antibody and/or T-cell immune response to protect a mammal from bacteria and viruses and related species and in the treatment or the prevention of Lyme diseases or tick encephalitis virus diseases.

Preferably, the pharmaceutical composition, the immunological composition, or vaccine, are used for the treatment or the prevention of various diseases especially in haematology (for the treatment or the prevention of cardiovascular diseases especially for improving coagulation clinics), transplantation (for immunosuppression control in autoimmune diseases, in graft rejection and allergy) in rheumatology (in particular, in the treatment or the prevention of inflammation), in cancer and in general treatment.

Another aspect of the invention relates to a method for treating or preventing a disease affecting a mammal, particularly to prevent or cure the transmission of pathogenous agents by tick, especially by *Ixodes ricinus,* or the symptoms of diseases induced by tick or pathogenous agents transmitted by tick, said method comprising the step of administrating to said mammal a sufficient amount of the pharmaceutical composition according to the invention or the immunological composition or vaccine according to the invention.

Preferably, an immunological response in a mammal is induced by inoculating the mammal with *I. ricinus* salivary gland polypeptide or epitope-bearing fragments, analogues, recombinant vector, outer-membrane vesicles or cells (attenuated or otherwise), adequate to produce antibody and/or T cell immune response to protect said mammal from bacteria and viruses which could be transmitted during the blood meal of *I. ricinus* and related species. Preferably, the *I. ricinus* salivary gland polypeptides used are those encoded by the cDNAs defined in the sequence listing.

The immunological composition or vaccine have any suitable formulation, and is preferably administered orally or parenterally (including subcutaneous, intramuscular, intravenous, intradermal injection). Suitable parenteral administration formulation include aqueous and non-aqueous sterile injection solutions which may contain anti-oxidants, buffers, bacteriostats and solutes which render the formulation isotonic with the blood of the recipient, and aqueous and non-aqueous sterile suspensions which may include suspending agents or thickening agents.

The pharmaceutical composition (especially a vaccine) may comprise this various elements of the invention in addition with one or more carrier molecules or one or more adjuvant molecules, anti-oxidants, buffer, bacterio status, solutes thickening agents or ions. Examples of carrier molecules are vectors comprising the polynucleotide sequence according to the invention for a transfection transformation of a cell or a carrier molecule which could be complexed or bounded to one or more of this element. For instance, the isolated protein or polypeptide according to the invention could be bounded to a carrier molecule, such as BSA or hemocyanine for improving its antigenic and immunogenic properties especially for obtaining an efficient vaccinal immune response (humoral and cellular immune response, especially a T-cell immune response).

Furthermore, the isolated nucleotide sequence according to the invention could be bounded to one or more promoter/activator sequence which allows a modulated expression of said nucleotide sequence into specific cells. Vector comprising the isolated nucleotide sequence according to the invention could correspond to plasmids or viral vectors, to cationic vesicles or to other lipid membranes such as liposomes.

This carrier molecules or vectors could be also used as adjuvant for inducing an efficient immune response in a patient especially when the pharmaceutical composition is a vaccine.

The term "adjuvant" has its usual meaning in the art of vaccine technology, i.e. a substance or a composition of matter which is not in itself capable of mounting a specific immune response against the antigen of the vaccine, but which is nevertheless capable of enhancing the immune response against the antigen. In other words, the combination of vaccination with antigen and adjuvant induces an immune response against the antigen which is stronger than that induced by the antigen alone.

Suitable carriers for administration of vaccines are well known in the art and can include buffers, gels, microparticles, implantable solids, solvents, other adjuvants or any other means by which the antigen of the vaccine can be introduced into a subject and be made sufficiently available to produce an immune response to the antigen.

Examples of others adjuvant molecules are saponine or suitable fractions thereof and lipopolysaccharides as described in the document EP 671 948, saponine fractions with one or more sterols present in specific formulation are described in the document WO 2007/068907 in addition.

Other examples of adjuvants are metallic salts, oil in water emulsion, lipid and/or derivative thereof, aminoalkyl glucosaminide phosphate, immunostimulotary oligonucleotides QS21 or combination thereof possibly in association with liposome described in the document WO 2006/123155 or US Patent 6 544 518.

An adjuvant composition may also comprise proteins from the yersinia genus as described in document WO 02/304 58.

An adjuvant could comprise also one or more carrier molecule(s), such as metallic salt particles (aluminium phosphate, aluminium hydroxide, calcium phosphate, magnesium phosphate, iron phosphate, calcium carbonate, magnesium carbonate, calcium sulphate, magnesium hydroxide or double salt like ammonium-iron phosphate, potassium, iron phosphate, calcium iron phosphate, calcium magnesium carbonate or a mixture of these salts or polyporous polymeric particles (such as microbeads or nanoparticles (as described in document WO 02/30458)).

An adjuvant could correspond also to an immuno stimulatory CpG oligo nucleotide, preferably CpG oligo nucleotide having a length between 15 and 45 nucleotides.

The pharmaceutical composition (vaccine) may also comprise other compounds which are used for enhancing the antigenicity or immunogenicity of active compounds by addition of immuno modulators on immuno adjuvants such as a cytokines, interferons, tumor necrosis factors, transforming growth factors, or colony stimulating factors preferably interleukin-2. The immunogenicity of the pharmaceutical composition (vaccine) could be also induced by an adequate immuno adjuvant which is preferably selected from the group consisting of block copolymer, ethylene copolymer, acrylic acid copolymer, an acrylic acid copolymer emulsion, a mineral oil emulsion or a mixture thereof, (such as squalen or squalane).

The pharmaceutical composition (vaccine) of the invention is of any suitable pharmaceutical form. Suitable solid or liquid pharmaceutical forms are, for example, granules, powders, pill, tablets, capsules, suppositories, syrups, emulsions, suspensions, creams, aerosols, drops or injectable solution in ampoule form, in whose preparation excipients and additives such as disintegrants, binders, coating agents, swelling agents, lubricants, flavorings, sweeteners or solubilizers are customarily used. In the particular case of a slow-release composition, the pharmaceutical composition may comprise a biocompatible matrix suitable for slow-release.

Regarding the pharmaceutical carrier, in general, the nature of the carrier will depend on the particular mode of administration being employed. For instance, parenteral formulations usually comprise injectable fluids that include pharmaceutically and physiologically acceptable fluids such as water, physiological saline, balanced salt solutions, or the like as a vehicle. For solid compositions, conventional non-toxic solid carriers can include, for example, pharmaceutical grades of mannitol, lactose, or magnesium stearate. In addition to biologically-neutral carriers, pharmaceutical compositions to be administered can contain minor amounts of non-toxic additives, such as wetting or emulsifying agents, preservatives, and pH buffering agents and the like.

The route of administration of the vaccine or pharmaceutical composition according to the present invention can be any suitable route of administration. It can be topical, intradermal, subcutaneous, oral, intravenous, parenteral, intra-peritoneal.

The medical regime is any suitable regime. Amounts and regimens for the administration of the vaccine or the pharmaceutical composition according to the present invention can be determined by those with ordinary skill in the clinical art of treating diseases associated with trypanosome.

The formulations may be presented, for example, in unit-dose or multi-dose containers, sealed ampoules and vials and may be stored in a freeze-dried condition requiring only the addition of the sterile liquid carrier immediately prior to use.

The vaccine formulation may also include adjuvant systems for enhancing the immunogenicity to the formulation, such as oil-in water systems and other systems known in the art. The dosage will depend on the specific activity of the vaccine and can be readily determined by routine experimentation.

The present invention will be described hereafter in reference to the enclosed figures presented as non limiting examples of the present invention.

### Brief Description of the drawings

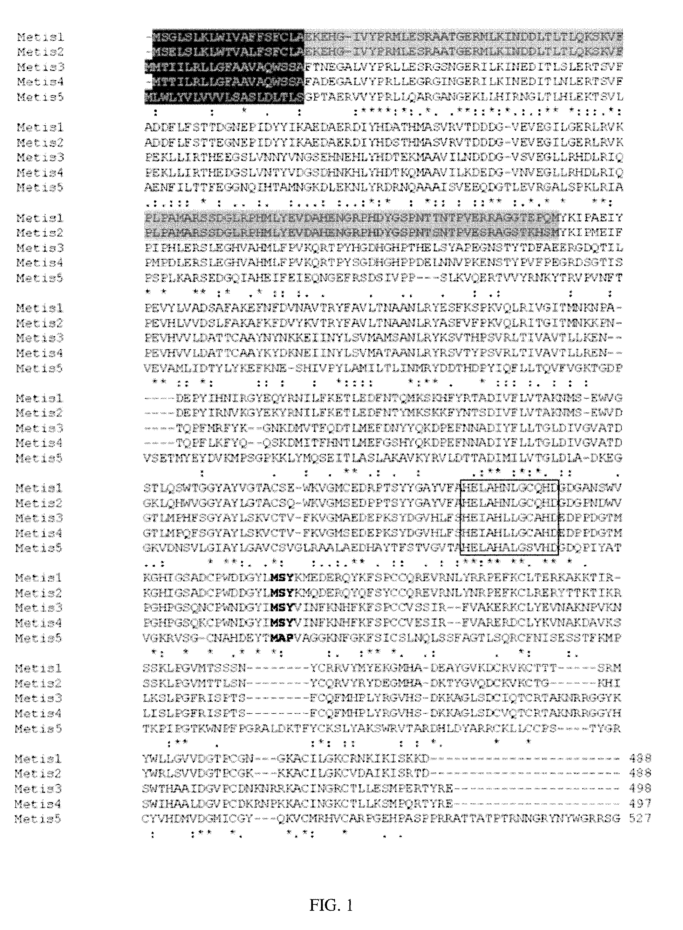

Figure 1 represents the alignment of the five Metis amino acid sequences. Amino acid sequences were predicted by *in silico* translation of the five ORFs found in the sequenced cDNAs. The predicted signal peptides are highlighted in reversed background. The predicted pro-sequences of Metis1 and Metis5 are highlighted in gray background. The zinc-binding motifs HEXXHXXGXXH/D are boxed. The putative methionine turn found after the zinc-binding domain is in bold. Asterisks indicate identities. Dots indicate similarities.

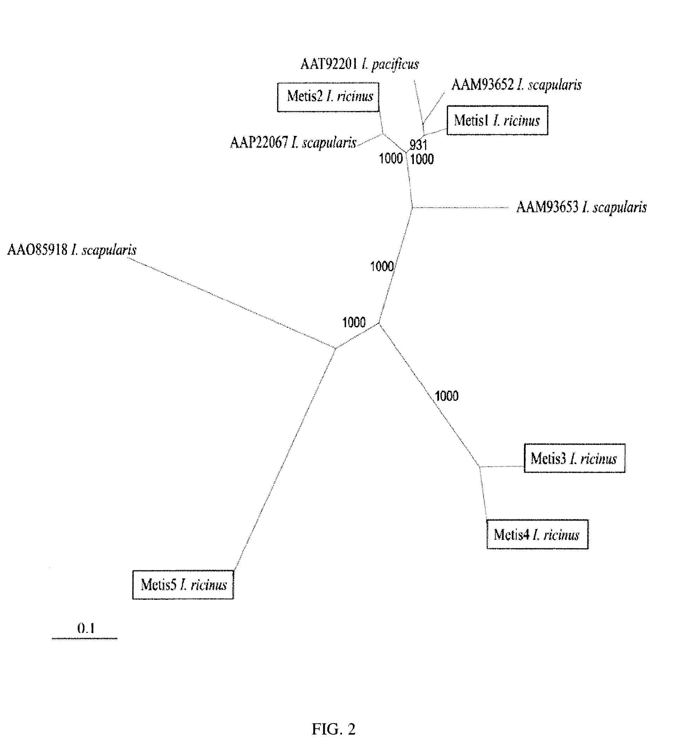

Figure 2 represents the phylogenic analysis of the metalloproteases from *I*. *ricinus, I. scapularis* and *I. pacificus*. Amino acid sequences predicted by *in silico* translation of the corresponding open reading frames were analyzed by the distance and neighbour-joining methods. The validity of the tree was verified by bootstrap analysis and the obtained bootstrap values are indicated at the branch nodes. Members of the Metis family are boxed.

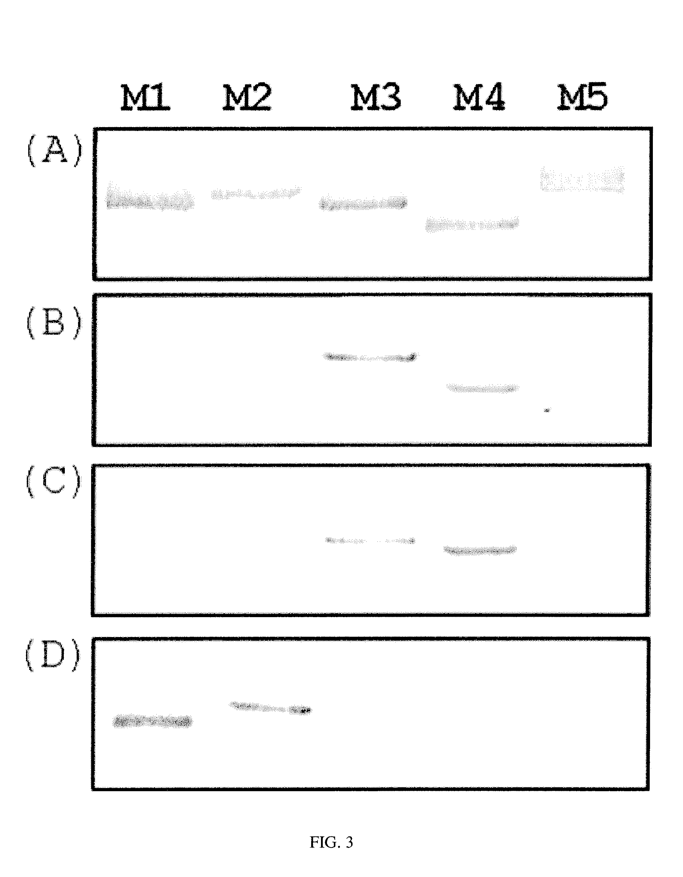

Figure 3 represents the antigenic specificity of the Metis proteins. The five pCDNA3.1/V5-His expression vectors for Metis1 to Metis5 (M1 to M5) were used for protein expression in COS-1 cells and genetic immunization of mice. Standardized amounts of the five Metis proteins harvested from culture supernatants were submitted to Western blot analysis using anti-V5 antibodies or sera from the immunized mice. Mice immunized with Metis2 and 5 did not raise detectable antibodies (A) Analysis with anti-V5 antibodies demonstrated equal loading of the 5 Metis (B) The anti-Metis3 serum only detected recombinant Metis3 and Metis4 (C) The anti-Metis4 serum only detected recombinant Metis3 and Metis4 and (D) the anti-Metis1 serum only recognized recombinant Metis1 and Metis2.

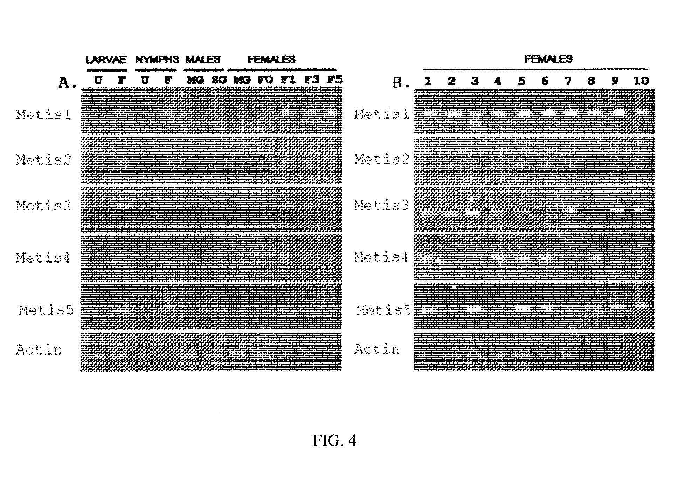

Figure 4 represents the stage, time and individual specific expression of Metis genes. Poly A(+) RNAs were extracted from different developmental stages, at different times of the blood meal, from different tissues and different individuals and analyzed by RT-PCR using pairs of primers specific for Metis1 to 5 and actin. PCR products were analyzed by agarose gel electrophoresis and ethidium bromide staining and sequenced. (A) Analysis of different tissues from different developmental stages at different steps of the blood meal from pooled populations. Midguts (MG), salivary glands (SG), salivary glands of unfed (F0), 1 day fed (F1), 3 day fed (F3), 5 day fed (F5) females or whole unfed (U) or fed (F) ticks. (B) Analysis of RNA extracted from salivary glands of 10 individual females (numbered 1 to 10) fed for 5 days.

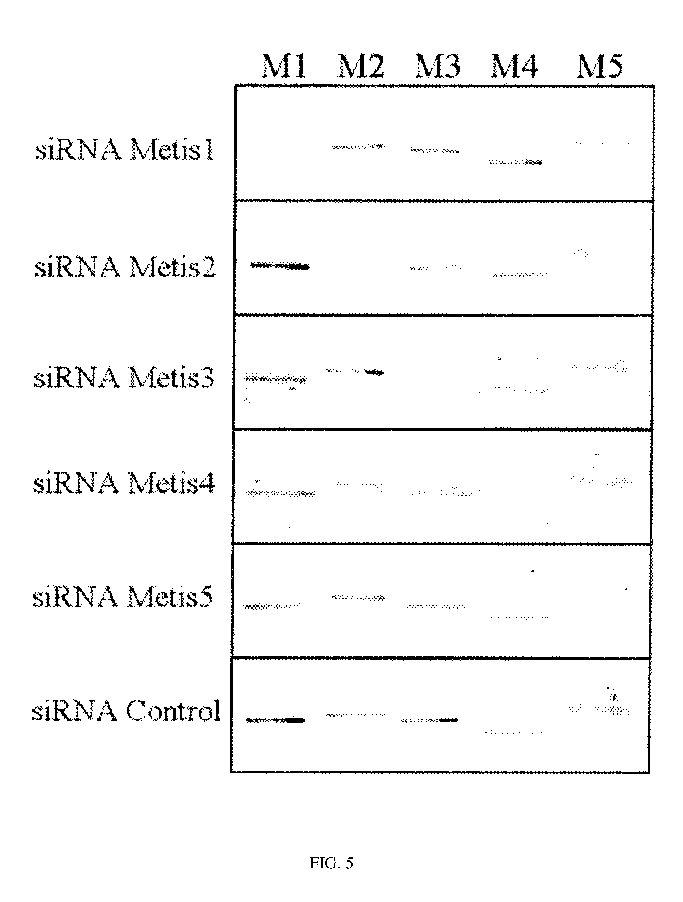

Figure 5 represents the specific siRNA silencing of *metis* gene expression. All possible combinations of each of the pCDNA3.1/V5-His vectors expressing Metis1 to Metis5 (M1 to M5) and each of the siRNA targeting *Metis1* to 5 (siRNA *metis1* to 5) were co-transfected in COS-1 cells and Metis protein expression was analysed by Western blot using anti-V5 antibodies.

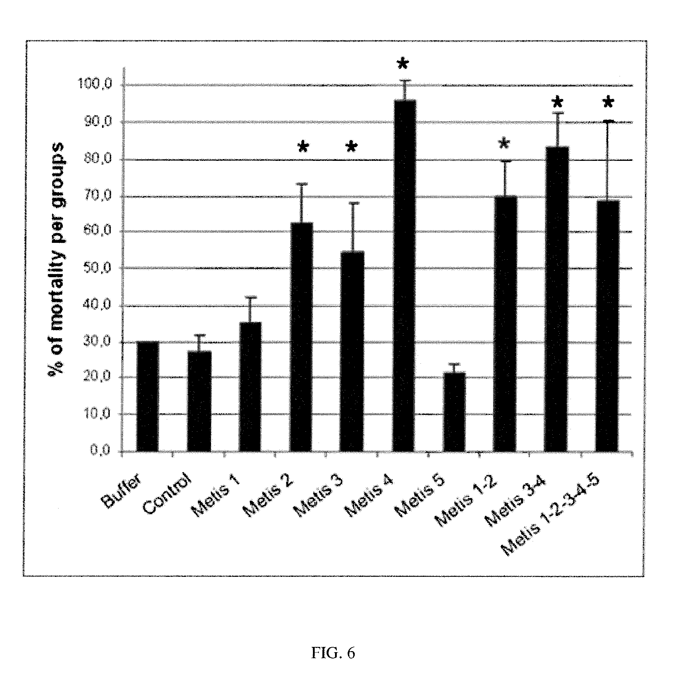

Figure 6 represents the tick viability after Metis knock down by RNA interference. *I. ricinus* adult ticks (30 ticks per experimental group) were injected into the idiosoma with buffer alone or siRNA targeting the 5 metis genes individually, grouped by two (e.g. *Metis1* and *Metis2, Metis3* and *Metis4*), or all 5 *metis* as well as siRNA targeting an irrelevant sequence (control). Ticks were followed daily until day 5 post infestation. The percentage of dead ticks and the standard deviation on 3 independent experiments are shown. * p<0.05 compared to control (Anova one-way of variance and Student-Newman-Keuls test).

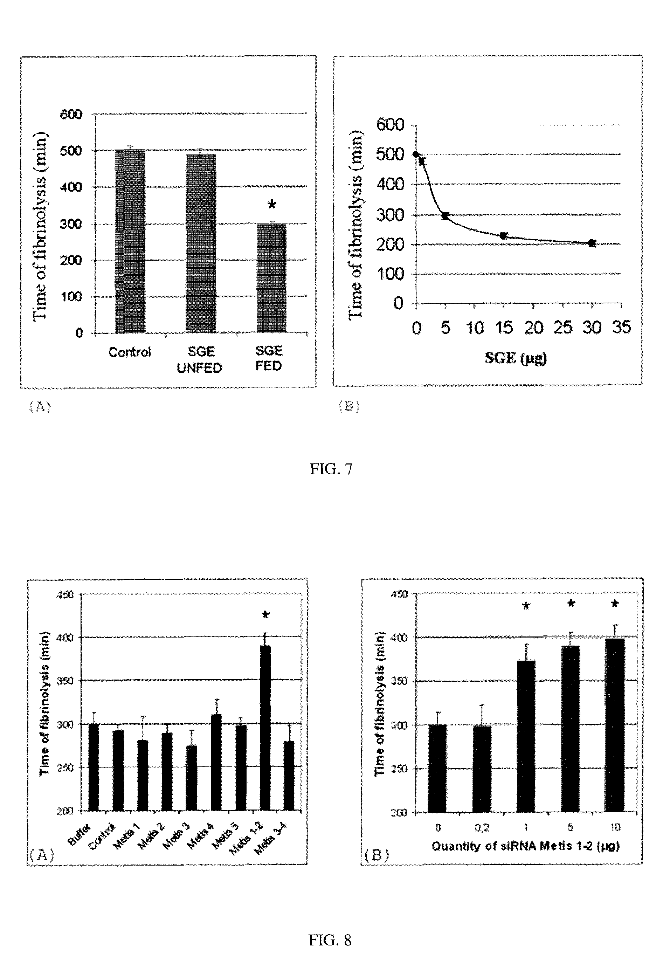

Figure 7 represents the fibrinolysis in presence of salivary glands extracts (SGE). (A) SGEs were incubated with a pre-formed fibrin clot. The time necessary for clot reduction was measured using the Euglobuline Clot Lysis time assay. 5µg of SGE from unfed and 5 day fed adult females or buffer alone was added to the fibrin clot. (B) The indicated amounts of 5 day fed adult female SGE were added to the fibrin clot. * p<0.05 when compared to the control (Anova one-way of variance and Student-Newman-Keuls test).

Figure 8 represents Metis knock down interfered with the stimulation of fibrinolysis by salivary gland extracts (SGE). (A) SGEs were incubated with buffer or the indicated siRNA for 6 hours prior to incubation with a pre-formed fibrin clot. The time necessary for clot reduction was measured using the Euglobuline Clot Lysis time assay. (B) SGEs were incubated with an increasing quantity of a combination of siRNA *Metis1* and *2* prior to incubation with a pre-formed fibrin clot. The time necessary for clot reduction was measured using the Euglobuline Clot Lysis time assay. * p<0.05 when compared to the control (Anova one-way of variance and Student-Newman-Keuls test).

### Definitions

« Polypeptide » refers to any peptide or protein comprising two or more amino acids joined to each other by peptide bonds or modified peptide bonds, i.e., peptide isosteres. "Polypeptide" refers to both short chains, commonly referred to as peptides, oligopeptides or oligomers, and to longer chains, generally referred to as proteins. Polypeptides may contain amino acids other than the 20 gene-encoded amino acids. "Polypeptides" include amino acid sequences modified either by natural processes, such as posttranslational processing, or by chemical modification techniques which are well known in the art. Such modifications are well described in basic texts and in more detailed monographs, as well as in a voluminous research literature. Modifications can occur anywhere in a polypeptide, including the peptide backbone, the amino acid side-chains and the amino or carboxyl termini. It will be appreciated that the same type of modification may be present in the same or varying degrees at several sites in a given polypeptide. Also, a given polypeptide may contain many types of modifications. Polypeptides may be branched as a result of ubiquitination, and they may be cyclic, with or without branching. Cyclic, branched and branched cyclic polypeptides may result from posttranslational natural processes or may be made by synthetic methods. Modifications include acetylation, acylation, ADP-ribosylation, amidation, covalent attachment of flavin, covalent attachment of a hem moiety, covalent attachment of a nucleotide or nucleotide derivative, covalent attachment of a lipid or lipid derivative, covalent attachment of phosphotidylinositol, cross-linking, cyclization, disulfide bond formation, demethylation, formation of covalent cross-linkings, formation of cystine, formation of pyroglutamate, formylation, gamma-carboxylation, glycosylation, GPI anchor formation, hydroxylation, iodination, methylation, myristoylation, oxidation, proteolytic processing, phosphorylation, prenylation, racemization, selenoylation, sulfation, transfer-RNA mediated addition of amino of amino acids to proteins such as arginylation, and ubiquitination. See, for instance, PROTEINS - STRUCTURE AND MOLECULAR PROPERTIES, 2nd Ed., T. E. Creighton, W. H. Freeman and Comany, New York, 1993 and Wolt, F., Posttranslational Protein Modifications: Perspectives and Prospects, pgs. 1-12 in POSTTRANSLATIONAL COVALENT MODIFICATION OF PROTEINS, B. C. Johnson, Ed., Academic Press, New York, 1983; Seifter et al., "Analysis for protein modifications and nonprotein cofactors", Meth Enzymol (1990) 182 : 626-646 and Rattan et al, "Protein Synthesis : Posttranslational Modifications and Aging", Ann NY Acad Sci (1992) 663 : 48-62.

"Polynucleotide" generally refers to any polyribonucleotide or polydeoxyribonucleotide, which may be unmodified RNA or DNA or modified RNA or DNA. "Polynucleotides" include, without limitation single- and double-stranded DNA, DNA that is a mixture of single- and double-stranded regions, single- and double- stranded RNA, and RNA that is a mixture of single- and double-stranded regions, hybrid molecules comprising DNA and RNA that may be single-stranded or, more typically, double-stranded or a mixture of single- and double-stranded regions. In addition, "Polynucleotide" refers to triple-stranded regions comprising RNA or DNA or both RNA and DNA. The term "Polynucleotide" also includes DNAs or RNAs containing one or more modified bases and DNAs or RNAs with backbones modified for stability or for other reasons. "Modified" bases include, for example, tritylated bases and unusual bases such as inosine. A variety of modifications has been made to DNA and RNA; thus, "Polynucleotide" embraces chemically, enzymatically or metabolically modified forms of polynucleotides as typically found in nature, as well as the chemical forms of DNA and RNA characteristic of viruses and cells. "Polynucleotide" also embraces relatively short polynucleotides, often referred to as oligonucleotides.

"Variant" as the term is used herein, is a polynucleotide or polypeptide that differs from a reference polynucleotide or polypeptide respectively, but retains essential properties. A typical variant of a polynucleotide differs in nucleotide sequence from another, reference polynucleotide. Changes in the nucleotide sequence of the variant may or may not alter the amino acid sequence of a polypeptide encoded by the reference polynucleotide. Nucleotide changes may result in amino acid substitutions, additions, deletions, fusions and truncations in the polypeptide encoded by the reference sequence, as discussed below. A typical variant of a polypeptide differs in amino acid sequence from another reference polypeptide. Generally, differences are limited so that the sequences of the reference polypeptide and the variant are closely similar overall and, in many regions, identical. A variant and reference polypeptide may differ in amino acid sequence by one or more substitutions (preferably conservative), additions and deletions in any combination. A substituted or inserted amino acid residue may or may not be one encoded by the genetic code. A variant of a polynucleotide or polypeptide may be a naturally occurring such as an allelic variant, or it may be a variant that is not known to occur naturally. Non-naturally occurring variants of polynucleotides and polypeptides may be made by mutagenesis techniques or by direct synthesis. Variants should retain one or more of the biological activities of the reference polypeptide. For instance, they should have similar antigenic or immunogenic activities as the reference polypeptide. Antigenicity can be tested using standard immunoblot experiments, preferably using polyclonal sera against the reference polypeptide. The immunogenicity can be tested by measuring antibody responses (using polyclonal sera generated against the variant polypeptide) against purified reference polypeptide in a standard ELISA test. Preferably, a variant would retain all of the above biological activities.

"Identity" or "sequence identity" is a measure of the identity of nucleotide sequences or amino acid sequences. In general, the sequences are aligned so that the highest order match is obtained. "Identify" *per se* has an art-recognized meaning and can be calculated using published techniques. See, e.g.: (COMPUTATIONAL MOLECULAR BIOLOGY, Lesk, A.M., ed., Oxford University Press, New York, 1988; BIOCOMPUTING : INFORMATICS AND GENOME PROJECTS, Smith, D.W., ed., Academic Press, New York, 1993; COMPUTER ANALYSIS OF SEQUENCE DATA, PART I, Griffin, A.M., and Griffin, H.G., eds, Humana Press, New Jersey, 1994; SEQUENCE ANALYSIS IN MOLECULAR BIOLOGY, von Heijne, G., Academic Press, 1987; and SEQUENCE ANALYSIS PRIMER, Gribskov, M. and Devereux, J., eds, M Stockton Press, New York, 1991). While there exist a number of methods to measure identity between two polynucleotide or polypeptide sequences, the term "identity" is well known to skilled artisans (Carillo, H., and Lipton, D., SIAM J Applied Math (1998) 48: 1073). Methods commonly employed to determine identity or similarity between two sequences include, but are not limited to those disclosed in Guide to Huge Computers, Martin J. Bishop, ed., Academic Press, San Diego, 1994, and Carillo, H., and Lipton, D., SIAM J Applied Math (1988) 48 : 1073. Methods to determine identity and similarity are codified in computer programs. Preferred computer program methods to determine identity and similarity between two sequences include, but are not limited to, GCG program package (Devereux, J., et al., J Molec Biol (1990) 215: 403). Most preferably, the program used to determine identity levels was the GAP program, as was used in the Examples hereafter.

As an illustration, by a polynucleotide or polypeptide having an amino acid or nucleotide sequence having at least, for example, 95% "identity" to a reference nucleotide or polypeptide sequence is intended that the sequence is identical to the reference sequence except that the sequence may include an average up to five point mutations per each 100 nucleotides or 30 amino acids of the reference sequence. In other words, to obtain a sequence at least 95% identical to a reference sequence, up to 5% of the nucleotides or amino acids in the reference sequence may be deleted or substituted with another nucleotide or amino acid or a number of nucleotides or amino acids up to 5% of the total nucleotides or amino acids in the reference sequence may be inserted into the reference sequence. These mutations of the reference sequence may occur at the 5' or 3' terminal positions of the reference nucleotide sequence or anywhere between those terminal positions, interspersed either individually among nucleotides in the reference sequence or in one or more contiguous groups within the reference sequence.

A "fragment" of a polypeptide is having an amino acid sequence that is the same as a part, but not all, of the amino acid sequence of the aforementioned *I. ricinus* salivary gland polypeptides of the invention. As with *I. ricinus* salivary gland polypeptides, fragment may be "free-standing" or comprised within a larger polypeptide of which they form a part or region, most preferably as a single continuous region. Preferred fragments include, for example, truncated polypeptides having the amino acid sequence of the *I. ricinus* salivary gland polypeptides of the invention, except for deletion of a continuous series of residues that includes the amino terminus, or a continuous series of residues that includes the carboxyl terminus and / or transmembrane region or deletion of two continuous series of residues, one including the amino terminus and one including the carboxyl terminus. Also preferred are fragments characterised by structural or functional attributes such as fragments that comprise alpha-helix and alpha-helix forming regions, beta-sheet and beta-sheet forming regions, turn and turn-forming regions, coil and coil-forming regions, hydrophilic regions, hydrophobic regions, alpha amphipathic regions, beta amphipathic regions, flexible regions, surface-forming regions, substrate binding region, and high antigenic index regions. Other preferred fragments are biologically active fragments. Biologically active fragments are those that mediate *I. ricinus* salivary gland protein activity, including those with a similar activity or an improved activity, or with a decreased undesirable activity. Also included are those that are antigenic or immunogenic in an animal or in a human.

### Detailed disclosure of the invention

In the document EP1187916, the inventors had characterised genes induced in the salivary glands of *Ixodes ricinus,* during the slow-feeding phase of the blood meal. Particularly, this document described five homologous genes coding for putative metalloproteases. These five genes were characterized in the follow-up of a differential screening between salivary gland cDNA libraries from fed and unfed *I. ricinus* female ticks. Particularly, the cloning of these genes was carried out by setting up two complementary DNA(cDNA) libraries. The first one was a subtractive library based on the methodology described by Lisitsyn et al. and improved by Diatchenko et al. This library cloned selectively induced mRNA during the tick feeding phase. The second library was a full-length cDNA library which has been constructed by using the basic property of mRNAs (presence of apolyA tail in its 3'end and the cap structure in its 5'end). This cDNA library permitted the cloning of full-length cDNAs, corresponding to some incomplete cDNA sequences deemed of interest, and identified in the subtractive cDNA library.

This initial screening allowed the cloning of twenty-seven partial cDNAs (called in EP1187916 seq 1 to seq 27) corresponding to transcripts induced during the blood meal of female *I. ricinus.* One of them, termed seq16, was homologous to snake venom metalloproteases of the reprolysin family (Leboulle et al., 2002). Oligonucleotides were then designed from these twenty-seven partial sequences and then pooled to screen a full length expression library which allowed the isolation of four sequences closely related to seq16, seq16 itself which was renamed Metis1 for **Met**alloprotease ***I**xodes ricinu**s**,* and a new sequence which was called Metis2. The forty-four clones obtained during this screening also contained three other sequences found to be more distantly related to seq16. Primers (table 1) were designed to clone the corresponding full-length cDNAs using 5' and or 3' RACE-PCR and the three new sequences were named Metis 3 to 5.

**Table 1: Primer sequences used for the screening of the full-length cDNA library**

| Clone | Probe (5'- 3') |
|---|---|
| seq1 | GTCCTCTTCTAAATAAGACCCATCC |
| seq2 | AAGTCACTTGCACTTATCAAGCTCC |
| seq3 | TTATGCTGCCGCCTACTTTTCCTTC |
| seq4 | AGTACCCCTGTGAACTCTGGCTTTG |
| seq5 | ATTGCCCTTGACGTACTCTCTCAAC |
| seq6 | GAAGGAACAGGCACAAATATACTAC |
| seq8 | GACCGATTCCACATTGTAGTACACC |
| seq9 | TGTGACCATATCTTTGTTTCCCCTG |
| seq10 | ACATATCATTTGGAGGAAGGCGTAG |
| seq11 | GTGATAACCATATCCATTCCTCACC |
| seq12 | TGGTTTACCGTAACAAGTACACCAG |
| seq13 | CTGCCTCTACAAAGTCAATGCCAAG |
| seq14 | CTCACCAAACACATCAAATACCCCC |
| seq15 | CATGCCTTCGTCGTACATATACACC |
| seq17 | TCGAATTGCACTTCGGAACTTCCAC |
| seq18 | TCCCCGCCCCTTGACAATCGTCCGA |
| seq19 | ATCCGAATGAGTTGTCAAATGACAT |
| seq20 | AGAAGAGTAAGGTTTTCACCGACAG |
| seq21 | TGTTGCTACAGACTCGACGTTTCGA |
| seq22 | TGAAACTTGAAATACTCCCACAGTC |
| seq23 | GACCACCCCGTCCGAACTTGCTAAA |
| seq25 | TCCAATCTACAATCTTTCCTCGCAC |
| seq26 | AGAAGACTGGGAAGATAAGAAGCAC |
| seq27 | TCACCTGCTATTCCAGAAGTACACC |

A bioinformatic analysis led to the following observations, illustrated in figure 1. All sequences contained a putative signal peptide (as determined by Signal P3.0). Sequence analysis and comparison with an already described *Ixodes scapularis* sequence (Francischetti et al., 2003) indicated the presence of a propeptide. The cleavage site was tentatively located between position 169 and 170 for the Metis1 and Metis2 proteins. A Zn binding domain was detected, which is characteristic of the M12B reprolysin family of the metzinin group. The belonging of Metis proteins to this family was further supported by a Met turn encoded 3' of the catalytic site.

The nucleotide sequences and the corresponding translated amino acid sequences of the five Metis members were used to construct a distance tree using clustalW software. The tree, presented in figure 2, was then submitted to bootstrap analysis. The inventors have also included in this tree all the putative metalloproteases from *Ixodes* species to date. This tree was supported by high bootstrap values. Inside this branch, Metalloproteases proteins clustered in four sub-branches. The first contained Metis3 and Metis4 which shared a 84.7% identity. The second contained Metis1 and Metis2 which shared a 84% identity. Metis5 and AA085918 from *I. scapularis* are not related to any cluster. The identity level between members of the different subgroups was only located between 13.7% and 36.4%.

Further analysis, detailed in table 2, indicated that the identity level between the different sub-branches of *Metis* was higher when considering nucleotide sequences than when considering amino-acid sequences. Therefore, the Nei-Gojobori method was used to calculate the number of synonymous changes per synonymous site (dS) and the number of non-synonymous changes per non synonymous sites (dN). Analysis of the values indicated at a higher ratio between non-synonymous and synonymous substitutions when comparing members of different phylogenic groups than in the same phylogenic group (Table 3).

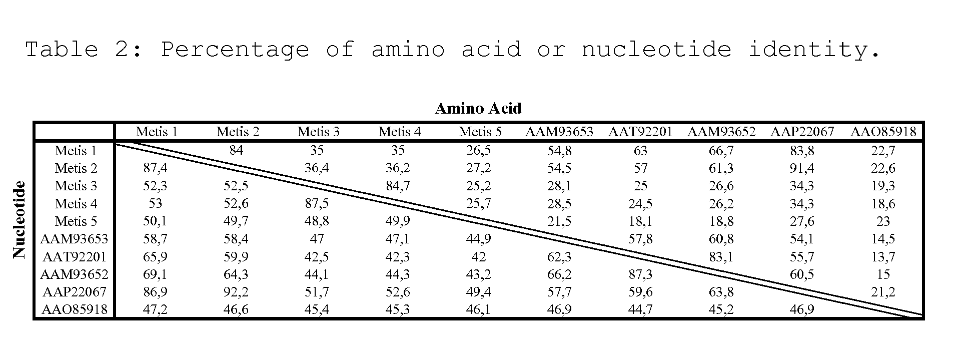

**Table 3: Divergence among Ixodes species. Pairwise estimates of synonymous (dS) and non synonymous (dN) substitutions per site by Modified Nei-Gojobori method (Nei and Gojobori, 1986).**

| dN | Metis1 | Metis2 | AAM93653 | AAT92201 | AAM93652 | AAP22067 | Metis3 | Metis4 | Metis5 | AA085918 |
|---|---|---|---|---|---|---|---|---|---|---|
| Metis1 | | 0,102 | 0,216 | 0,059 | 0,063 | 0,1 | 0,774 | 0,486 | 0,566 | 0,649 |
| Metis2 | | | 0,215 | 0,126 | 0,113 | 0,05 | 0,48 | 0,487 | 0,56 | 0,573 |
| AAM93653 | | | | 0,226 | 0,23 | 0,225 | 0,502 | 0,511 | 0,568 | 0,686 |
| AAT92201 | | | | | 0,064 | 0,129 | 0,495 | 0,489 | 0,579 | 0,717 |
| AAM93652 | | | | | | 0,114 | 0,492 | 0,496 | 0,573 | 0,694 |
| AAP22067 | | | | | | | 0,489 | 0,496 | 0,56 | 0,589 |
| Metis3 | | | | | | | | 0,083 | 0,583 | 0,601 |
| Metis4 | | | | | | | | | 0,573 | 0,604 |
| Metis5 | | | | | | | | | | 0,535 |
| AA085918 | | | | | | | | | | |
| | | | | | | | | | | |

| dS | Metis1 | Metis2 | AAM93653 | AAT92201 | AAM93652 | AAP22067 | Metis3 | Metis4 | Metis5 | AA085918 |
|---|---|---|---|---|---|---|---|---|---|---|
| Metis1 | | 0,187 | 0,45 | 0,105 | 0,121 | 0,201 | 0,619 | 0,594 | 0,559 | 0,583 |
| Metis2 | | | 0,455 | 0,238 | 0,231 | 0,144 | 0,609 | 0,612 | 0,588 | 0,603 |
| AAM93653 | | | | 0,43 | 0,437 | 0,449 | 0,632 | 0,633 | 0,611 | 0,626 |
| AAT92201 | | | | | 0,437 | 0,449 | 0,618 | 0,653 | 0,613 | 0,6 |
| AAM93652 | | | | | | 0,242 | 0,638 | 0,64 | 0,598 | 0,603 |
| AAP22067 | | | | | | | 0,616 | 0,592 | 0,575 | 0,628 |
| Metis3 | | | | | | | | 0,224 | 0,645 | 0,591 |
| Metis4 | | | | | | | | | 0,615 | 0,606 |
| Metis5 | | | | | | | | | | 0,594 |
| AA085918 | | | | | | | | | | |
| | | | | | | | | | | |

| dN/dS | Metis1 | Metis2 | AAM93653 | AAT92201 | AAM93652 | AAP22067 | Metis3 | Metis4 | Metis5 | AA085918 |
|---|---|---|---|---|---|---|---|---|---|---|
| Metis1 | | 0,545 | 0,480 | 0,562 | 0,521 | 0,498 | 0,774 | 0,818 | 1,013 | 1,113 |
| Metis2 | | | 0,473 | 0,529 | 0,489 | 0,347 | 0,788 | 0,796 | 0,952 | 0,950 |
| AAM93653 | | | | 0,526 | 0,526 | 0,501 | 0,794 | 0,807 | 0,930 | 1,096 |
| AAT92201 | | | | | 0,610 | 0,510 | 0,801 | 0,749 | 0,945 | 1,195 |
| AAM93652 | | | | | | 0,471 | 0,771 | 0,775 | 0,958 | 1,151 |
| AAP22067 | | | | | | | 0,794 | 0,838 | 0,974 | 0,938 |
| Metis3 | | | | | | | | 0,371 | 0,904 | 1,017 |
| Metis4 | | | | | | | | | 0,932 | 0,997 |
| Metis5 | | | | | | | | | | 0,901 |
| AA085918 | | | | | | | | | | |

Thus, the calculated dN/dS ratio was between 0.371 and 0.61 between members of the same cluster and 0.774 and 1.151 between members of different clusters. This bias towards non-synonymous substitutions strongly suggests that a positive Darwinian selection was at play and drove this evaluative divergence.

Pairs of primers were designed to RT-PCR amplify the five *Metis* Open Reading Frames (ORFs) from 5-day fed tick salivary glands mRNA used as a template (table 4).

**Table 4 :Primers used for amplification of the metis genes.**

| **Gene** | Direct Primer (5'-3') | Reverse Primer (5'-3') |
|---|---|---|
| *metis1* | GGATCCATGTCGGGACTCAGCCTGAAA | TTCGAAGTCCTTCTTGCTTATTTTGATTTT |
| *metis2* | GGTACCATGTCGGAACTCAGCCTGAAAT | TCTAGAGTCCGTTCTGCTTATTTTGATCG |
| *metis3* | GGTACCATGACTATCATTTTGCGGCTCC | TCTAGATTCCCGGTACGTTCTCTCCG |
| *metis4* | GGTACCATGACTACCATTTTGCGGCTCC | TCTAGATTCCCGGTACGTTCTCTGGG |
| *metis5* | GGTACCATGTTGTGGCTCTATGTGTTGGT | TCTAGAGCCACTTCGACGTCCCCAGT |

Unique restriction sites were added in 5' and 3' of the *Metis* ORFs by PCR in order to subclone them into the mammalian expression vector pCDNA3.1/V5-His coding for the His and V5 epitope tags.

The five *Metis* Open Reading Frame (ORF) were cloned into the pcDNA3.1/V5-His mammalian expression vector and constructs were transfected in COS-1 cells for transient expression. The ability of these vectors to drive the expression of whole V5-His fusion proteins was checked by Western blot analysis using an anti-V5 antibody (Figure 3A). These five constructs were then used for genetic immunization of mice.

The resulting sera were tested by Western blot analysis of the corresponding Metis proteins expressed in COS cells. These results showed that antibodies were raised only in mice immunized with pcDNA3.1/V5-His Metis1, Metis3 and Metis4 and not in mice immunized with pcDNA3.1/V5-His Metis2 and Metis5 (results not shown). Each of these sera was then used in Western blot analysis on each of the five recombinant proteins.

As shown in figure 3B to D, the anti-Metis3 and anti-Metis4 sera specifically recognized Metis3 and Metis4 respectively. However, while the anti-Metis1 reacted with Metis1 and cross reacted with Metis2. None of the immune sera recognized Metis5. These results suggest that the three Metis phylogenic groups display epitopes which are shared inside each of them but different between them.

The previous RT-PCR analysis of poly A+ RNA confirmed that the levels of Metis1 and Metis2 mature RNA was higher in salivary gland extracts of five day fed females as compared to unfed female ticks (data not shown). In order to extend this analysis to all the members of the Metis family at different blood meal steps, parasitic stages or individuals, specific pairs of primers (table 5) were designed, able to discriminate between the different members of the family and amplify only one of them at a time (data not shown). A pair of primers (sense-primer; 5'-ATGTGTGACGACGAGGTTGCC-3' and anti-sense primer; 5'-TTAGAAGCACTTGCGGTGGATG-3') allowing actin mRNA amplification was used in order to verify the integrity and correct loading of the samples.

**Table 5: Primers used for specific amplification of the Metis fragments.**

| **Gene** | Direct Primer (5'-3') | Reverse Primer (5'-3') | Size (pb) |
|---|---|---|---|
| *metis1* | GTCAAAACATTTTTATCGTACTGCC | GATTGTTTTTTTCGCTTTTCGTTCAG | 423 |
| *metis2* | ATCAAAAAAATTCTATAATACCTCCGAT | TATTGTTTTTGTCGTGTATCGTTCTC | 423 |
| *metis3* | GCTTGACATAGTTGGGGTAGCGA | TTCCTCCTGTTTTTGTTGTCGCAA | 588 |
| *metis4* | GCTTGATATAGTTGGGGTAGCGA | TTCTTGGGGTTTCTCTTGTCGCAA | 588 |
| *metis5* | ATGATGAATATACTATGGCGCCTGT | GTCCATTATTACGCGTCGGGGTT | 456 |

In order to monitor time, tissue and individual specific expression, these pairs of primers were used to analyze polyA(+) RNA extracted from various sources: whole pooled unfed or fed larvae and nymphs, midguts and salivary glands from males, midguts from females as well as salivary glands from unfed and one, three or five day-fed females, and finally from salivary glands of ten individual females fed for five days.

Although the RT-PCR illustrated in figure 4 was only semi-quantitative, it allowed to draw the following conclusions: First, none of the Metis transcripts were detectable in the salivary glands of adult males; Second, none of the Metis transcripts were detectable in the guts of adult males or females; and third, expression of the five Metis genes were not detectable in unfed ticks. However, the expression of the five Metis genes was induced upon blood feeding in larvae, nymphs and females. A finer analysis of the females indicated that the expression was induced specifically in salivary glands as early as the first day of a blood meal. It also indicated that amongst a tick population, the pattern of expression of the five metalloproteases varied amongst different individuals. These results suggest that the Metis genes are induced and expressed specifically in salivary glands during a blood meal.

In order to investigate the role of the Metis family, we decided to observe ticks upon *in vivo Metis* gene knock down by RNAi. siRNAs specific for each member of the family were designed (Table 6) and their specificity assessed by co-transfection with each of the pcDNA3.1 expression vectors in COS-1 cells.able 6: siRNA used for silencing of the *metis* genes.

| **Gene** | Direct siRNA (5'-3') | Reverse siRNA( 5'-3') |
|---|---|---|
| *metis1* | ACACUCAGAUGAAGUCAAA | UUUGACUUCAUCUGAGUGU |
| *metis2* | GCACGACAGAGGGAAAUGA | UCAUUUCCCUCUGUCGUGC |
| *metis3* | CAACAUGCGCUGCUUACAA | UUGUAAGCAGCGCAUGUUG |
| *metis4* | CGAGGAGGCUACCAUUCAU | AUGAAUGGUAGCCUCCUCG |
| *metis5* | CUACUGCGGAUAUAAUGAU | AUCAUUAUAUCCGCAGUAG |

Supernatants of all the co-transfections were harvested and submitted to Western blot analysis using anti-his antibodies.

Figure 5 shows that the expression of a given Metis protein was suppressed only when its expression vector was co-transfected with the matching siRNA but not in any other co-transfection. These results showed that each designed siRNA was functional as it knocked down the expression of the corresponding protein and highly specific as it did not affect the expression of other family members.

An analysis of the *in vivo* effect of each of these siRNA was performed. For that purpose, groups of thirty adult female ticks were microinjected with different siRNA, alone or pooled, in the ventral torso of the idiosoma.

As shown in figure 6, injection of the buffer alone or of irrelevant RNA as controls caused a 30% and 27.3 % mortality rate, respectively. Injection of each of the five siRNA targeting Metis1 to Metis5 alone had different effects on the basal mortality rate, ranging from a total lack of effect (Metis1 and Metis5) to a dramatic effect (Metis4) with intermediate effects for Metis2 and Metis3. This mortality was observed to be an early effect (within 12 hours). The absence of effect of some individual RNAi (together with the organization of the family in divergent phylogenic subgroups) suggests a possible functional redundancy. In order to check this hypothesis, the siRNAs targeted at all the members of one phylogenic branch at a time were pooled and injected. While we confirmed that the Metis5 siRNA had no effect, pooled Metis1 and Metis2 and pooled Metis3 and Metis4 induced a mortality of injected ticks of 70.0% and 83.3% respectively. The latter value is not significantly different from the value obtained after injection of Metis4 siRNA alone, which was already nearly completely letal on its own. The former suggests that the two members of the Metis1-2 group have redundant actions, as the combined action of the two siRNAs together is significantly stronger than each one alone. Finally, injection of a pool of the five siRNA gave a mortality rate of 68.9%. Although not significantly different, this lower value could be related to the fact that the amount of each siRNA in the cocktail is 5 times lower than in individual injections. These results suggest that some Metis proteins but not others are essential for the survival of ticks during a blood meal. Finally, no difference between control ticks and ticks surviving the microinjection could be observed by any of the analyzed criteria (weight gain, meal duration).

The mechanism affected by the action of Metis was investigate. As the expression of an active form of any of the Metis proteins is difficult to performed - a common problem encountered while studying metalloproteases and which prevents a direct assessment of enzymatic activity - an *ex vivo* siRNA interference assay was set up in order to bypass this problem.

Salivary gland extracts or purified proteins have been shown to interfere with several physiological processes *in vitro*. In particular, an effect on fibrinolysis has been observed, and it has been proposed to be mediated by metalloproteases; Francischetti et al., 2003). siRNAs were therefore mixed with the salivary gland extracts from dissected ticks and their influence was monitored in a fibrinolysis assay. In this assay, 5 µg of salivary gland extracts (SGE) from five day fed female ticks were mixed with a preformed fibrin clot and the fibrinolysis monitored by euglobin clot lysis time. In these conditions, the SGE from fed ticks decreased the fibrinloysis time by 206 ± 11 min (corresponding to 41% of the time observed with plasma only- in a dose dependent manner with a maximum of 301 ± 10 min (corresponding to 60%) for 30 µg of salivary gland extracts) as opposed to ungorged tick salivary extracts which did not influence the fibrinolysis time (figure 7).

As shown in figure 8A, none of the individually added Metis siRNA had an effect. Neither did the combination Metis3-4. On the contrary, the combination Metis1-2 siRNA added to salivary gland extracts interfered with their ability to decrease fibrinolysis time, as they raise it by half. That is to say, salivary gland extracts supplemented with buffer or a control siRNA lower the fibrinolysis time by 201 ± 14 min (40.1%) and by 208 ± 6 min (41.5%) respectively. Addition of pooled Metis1 and 2 siRNA reduced this action to 112 ± 16 min (22,4%). Addition of an equivalent amount of buffer or siRNA targeting other Metis or control RNA did not influence the measured values.

The specific action of the Metis1-2 siRNA was strongly supported by an observed dose dependent effect (see figure 8B) which however, nevertheless plateaued at high concentrations.

Among the reported roles of metalloproteases is the alteration of tissues, and in particular the remodelling of the basal lamina and extra cellular matrix. This has direct implications when saliva proteins of biting animals are considered. The toxicity of some snake venoms is due to the action of metalloproteases.

Several metalloprotease genes in the tick genome are already known and several reports on the sialome of *Ixodes scapularis* and *Ixodes pacificus* (Valenzuela et al., 2002; Francischetti et al., 2005; Ribeiro et al., 2006) indicate that organization in gene families is a recurrent theme in the salivary glands of these organisms.

An analysis of the amino acid sequences of five putative metalloproteases from the salivary glands of the tick *I. ricinus* (Metis protein family) revealed the following characteristics: a signal peptide, a Zinc binding domain, a conserved critical methionine and a pattern of C terminal cysteines, indicating that they are members of the metzinins group which include the snake venom proteases. The biochemical analysis performed was in agreement with a putative protease activity, as the knock down of some Metis genes' expression interfered with blood meal completion *in vivo* and modulated fibrinolysis by SGE *ex vivo.* These observations suggest that Metis-stimulated fibrinolysis, an action that counteracts haemostasis, interferes with wound healing of the endothelial epithelium and therefore facilitates the blood meal. It was no possible to rule out that at least some Metis (for example Metis4) are proteases with more widespread roles, such as digestion of food. This is supported by the lack of effect of Metis4 knock down on fibrinolysis. As Metis4 is essential for tick survival, this argues for the acquisition of a new function by this member of the family.

Although the saliva of some tick species has been shown to display a fibrino (geno)lytic action (Francischetti et al., 2003), only a few molecules of tick saliva have been described to interfere with the physiological process of fibrinolysis. One of the only examples is TCI (tick carboxypeptidase inhibitor) from *Rhipicephalus bursa,* which binds and inhibits TAFI (thrombin activable fibrinolysis inhibitor) to stimulate fibrinolysis (Arolas et al., 2005). However, some snake venom metalloproteases have been found to have saliva calcium dependent hydrolytic activity (Francischetti et al., 2003).

The sequence comparisons and phylogenic tree indicate that the Metis proteins belong to three subgroups. The observed bias towards non-synonymous substitutions strongly suggests that a positive Darwinian selection is at play, driving apart the members of these different Metis subgroups. This indicates an evolutive pressure towards functional diversification between these different subgroups. Several important evolutive problems, such as functional, developmental or tissue specialization, host range extension and antigenic variation, can be solved by diversification.

Regarding the functional specialization, the experiments described in the present invention indicate that there could be some redundancy inside given subgroups, as some members alone were dispensable for the blood meal to occur whilst at least one member of the two subgroups seemed necessary. At least one member (Metis4) seemed to be essential as the lack of this protein did not interfere with fibrinolysis at all. The diversification observed could have provided this protein with new unrelated functions indicating functional diversification. These observations were reminiscent of the few other reports investigating similar tick salivary proteins involved either in the same function (madanin) (Iwanaga et al., 2003) or not (Salp14 and Salp9pac) (Narasimhan et al., 2002) .

### Examples:

Biological materials used in this study:
*I. ricinus* ticks were bred and maintained at the Institute of Biology, University of Neuchâtel (Switzerland). Founders of the colony were initially collected in the woodland near Neuchâtel and have been maintained on rabbits or swiss mice for over 20 years. For the experiments described in this paper, pairs of adult (one female and one male) ticks were allowed to anchor and feed on rabbits for the indicated periods. The larvae and the nymphs were fed on mice until repletion. For the preparation of midgut or salivary gland extracts (SGE), unfed and 5 day engorged female ticks were dissected under the microscope. Salivary glands and midguts were harvested in ice-cold phosphate saline buffer (PBS). Tissues were then disrupted and homogenized using a dounce. Samples were centrifuged for 5 min. at 10,000g. Supernatants were recovered and stored at -20°C. For the preparation of larvae and nymphs extracts, whole fully engorged individuals were crushed and were prepared as above.

### Example 1: cDNA library Screening and rapid amplification of cDNA ends (RACE).

Analysis of a cDNA subtractive library from 5 day fed and unfed *I. ricinus* salivary glands originally identified twenty-seven distinct sets of sequences specific of the fed state (Leboulle et al., 2002). Specific oligonucleotides (described in table 1) were designed from these sequences, pooled and used to screen a full-length cDNA library by conventional hybridisation procedures. This screening identified fourty-four clones. These clones were sequenced using M13 forward and reverse primers. Sequences were BLASTed against the original twenty-seven clones and five sequences showed homology with a snake venom metalloprotease. In cases where full-length cDNA clones were not recovered, the missing ends were generated by rapid amplification of cDNA ends (RACE) using the GeneRacer Kit (Invitrogen).
In these cases gene-specific primers used for 5'-RACE were: *metis3:* 5'-TGACGTCCTCTCAAGGCTGAGGGTAA-3', *metis4: 5'-*TTGTTATGGTCCGAGCCGTCGACATAA-3', *metis5:* 5'-GGGGCTCAGTGCTCCTCTCACTTCTAA-3' ; and 3'-Race was : *metis2:* 5'-GCTAATCTTAGGTATGCCAGCTTCGTAT-3'. The amplification was made using ThermoZyme (Invitrogen) DNA Polymerase according to the manufacturer's instruction. Products were cloned into the pCR4-TOPO vector (Invitrogen) and sequenced.

### Example 2: RNA extraction and RT-PCR analysis

mRNA were isolated by oligo-dT chromatography (MicroFastTrack 2.0 mRNA Isolation Kit, Invitrogen) from various tissues at different developmental stages or from whole individuals after tissue disruption using a dounce homogenizer and clearing by centrifugation. Reverse transcription was routinely performed in a 20 µl standard RT reaction mixture according to the instructions of the manufacturer (First-Strand cDNA Synthesis System, Invitrogen) using the oligo dT primer. The RT product was then used as a template in 50 µl of a standard PCR reaction mixture with gene-specific primers described in Table 2 to generate products of the indicated size. Thus, the PCR was routinely performed in a 50 µl volume of Takara buffer containing 2.5 U of Taq polymerase (Takara Ex Taq, Takara, Japan), 10 pmoles of each primer, and 2.5 nmoles of each dNTP (Takara). PCR conditions were 30 cycles of 30 s at 95°C / 30 s at 58°C / 1 min at 72°C preceded by an initial 4 min denaturation step at 95°C and followed by a final 10 min extension step at 72°C. A pair of primers designed to amplify a 1131 bp from the actin complete ORF (sense-primer; 5'-ATGTGTGACGACGAGGTTGCC-3' and anti-sense primer; 5'-TTAGAAGCACTTGCGGTGGATG-3') was used as control. Ten µl of the PCR reactions were analyzed on a 2% agarose gel. PCR products were then cloned into the pCRII-TOPO vector (Invitrogen) and sequenced.

### Example 3: Expression of recombinant proteins in mammalian cells.

Pairs of primers were designed to RT-PCR amplify the 5 *metis* ORFs from 5-day fed tick salivary glands mRNA used as a template (see table 3). Unique restriction sites were added in 5' and 3' of the *metis* ORFs by PCR in order to subclone them into the mammalian expression vector pCDNA3.1/V5-His coding for the His and V5 epitope tags. The PCR were performed in a 50 µl reaction volume containing 2.5 U of Taq polymerase (Takara Ex Taq, Takara, Japan), 10 pmoles of specific primers for each target region amplified (Table 2), and 2.5 nmoles of each dNTP (Takara) in a standard buffer supplied by the manufacturer (Takara). The PCR conditions were 1 cycle at 95°C for 4 min followed by 30 cycles at 95°C for 30 s/ 58°C for 30 s/ 72°C for 2 min followed by a final extension step at 72°C for 10 min. The PCR products were then purified by polyacrylamide gel electrophoresis followed by electroelution. The PCR products were cloned into the pCDNA3.1/V5-His KpnI and *Xba*I (for *metis2* to *metis4*) or *Bam*HI and *Sfu*I (for *metis1*) restriction sites. The *metis*pCDNA3.1/V5-His constructs were then transfected into COS-1 cells by means of Fugene 6 (Roche) according to the manufacturer's recommendations. The expression of proteins recovered in the culture supernatant was confirmed by Western blot analysis.

### Example 4: SDS-PAGE and Western blot analysis

After protein dosage, recombinant proteins or tissue samples were mixed with one volume of 2X sample buffer, loaded on a 12.5% polyacrylamide gel and electrophoresed. Proteins were then analysed by Western blotting using the indicated primary antibodies, and AP coupled anti-rabbit secondary antibodies (Promega). Membranes were developed using 4-nitrobluetetrazolium (NBT) and 5-bromo-4-chloro-3- indolyl-phosphate (BCIP).

### Example 5: DNA immunization

Intramuscular injections of 100 µg *metis*/pCDNA3.1/V5-His in 100 µl PBS were performed in the quadriceps of mice and repeated five times at 3-week intervals. They were immediately preceded by a local injection of 100 µl cardiotoxin (10µM).

### Example 6: siRNA

siRNA (table 4) were designed to target specifically each of the *metis* mRNA and were synthesized by Eurogentec, Belgium.

### Example 7: siRNA silencing in COS-1 cells

COS-1 cells were co-transfected with all possible combinations of 500 ng of each of the *metis*/pCDNA3.1/V5-His and 100 ng of each of the specific siRNA using the X-tremeGENE siRNA transfection Reagent (Roche) according to the manufacturer's recommendations. 48 hours post transfection, culture supernatant was harvested and the protein expression was analyzed by Western blot

### Example 8: siRNA interference in live ticks.

1 µl of 1 mM EDTA, 10 mM Tris-HCl, pH 7.5 buffer only or containing 650 ng of specific *metis* siRNA or control siRNA duplexes (Eurogentec, Belgium) was injected into the ventral torso of the idiosoma, away from the anal opening of adult *I. ricinus* females. The injections were carried out using Hamilton Microliter syringes with 33-gauge needles. Thirty ticks were used per group. The ticks were allowed to recover for one day before infestation (together with uninjected male ticks) of the ears of New Zealand White rabbits. The ticks were monitored daily. Female ticks that fell off upon repletion were collected and weighed on a digital balance.

### Example 9: ex vivo siRNA silencing in salivary glands extracts.

The salivary glands from ten partially (5 days) fed female ticks were incubated for 6 h at 37°C in presence of 5 µg of the control siRNA duplexes (Eurogentec, Belgium) or *metis* siRNA (individually or in combination) or buffer alone in a total volume of 200 µl of incubation buffer TC-199 (Sigma) containing 20 mM MOPS, pH 7.0.

### Example 10: Fibrinolysis assay: Euglobulin Clot Lysis Time (ECLT).

The euglobulin fraction was prepared as described by Zouaoui Boudjeltia (2002). Briefly, 75 µl of acetic acid (0.025%) and 900 µl of deionised water were added to 100 µl of human plasma. The sample was centrifuged at 4000g for 10 min at 4°C. The pellet was resuspended in 100 µl of Owren buffer. Euglobulin clot formation was started by adding 25 µl (1.5 U/ml) of thrombin. After 10 min , 10 µl of SGE in Owren buffer were added to the reaction and the lysis time was measured by a semiautomatic method using a "Lysis Timer" device .

### Example 11: Bioinformatics.

For sequence collection, the GenBank databases were interrogated online at the NCBI server using the Blast program. Nucleotide and amino acid sequences of Metis were used as baits to screen the species databases or genome projects preliminary releases. Translation of the open reading frame was performed using the Expasy software. Alignments were performed and visualised using the ClustalW software.
Signal peptide and its cleavage site were determined on the amino-acid sequences using the signalP 3.0 software. Calculations of molecular weights were performed using the pepstat software. The inframe alignment of the codons was obtained by aligning the coding sequences using ClustalW under the Mega3 package. Manual adjustments were made. The genedoc package was then used to visualise alignments. Identity levels were also calculated using the genedoc package. Phylogenic analysis was performed using the distance and neighbour joining methods. We performed the bootstrap analysis of 1000 replicates of the original datasets; trees were visualised using the Tree View software. The dN and dS values were calculated using the Nei-Gojobori method as implemented in the Mega3.1 package.

### Example 12: Statistical Analyses

The MedCalc statistical software was used for statistical analysis of the results.

### References

Anguita, J., et al. Immunity 16,849-59. 2002.
Arolas, J.L., et al. J. Biol. Chem. 280, 3441-8. 2005.
Boldbaatar, D., Insect Biochem. Mol. Biol. 36, 25-36. 2006.
Diatchenko et al. Proc. Natl. Acad. Sci. USA 93, 6025-6030. 1996.
Francischetti, I.M., et al. Blood 99, 3602-12. 2002.
Francischetti, I.M., et al. Biochem. Biophys. Res. Commun. 305, 869-75. 2003.
Francischetti, I.M., et al. Thromb. Haemost. 94, 167-74. 2005.
Francischetti, I.M., et al. Insect. Biochem. Mol. Biol. 35, 1142-61. 2005.
Gentz et al. 1989, Proc Natl Acad Sci USA, 86:821-824.
Iwanaga, S., et al. Eur. J. Biochem. 270, 1926-34. 2003.
Kato, N., et al. Thromb. Haemost. 93, 359-67. 2005.
Kohler and Milstein, Nature(1975) 256, 495 .
Law, J.H., et al. Annu. Rev. Biochem. 61, 87-111. 1992.
Leboulle, G., et al. J. Biol. Chem. 277, 10083-9. 2002.
Leboulle, G., et al. Am. J. Trop. Med. Hyg. 66, 225-33. 2002.
Lisitsyn et al. Science 259,946-951. 1993.
Nei, M. and Gojobori, T., Mol. Biol. Evol. 3, 418-26. 1986.
Narasimhan, S., Insect Mol. Biol. 11, 641-50. 2002.
Paesen, G.C., Mol. Cell. 3, 661-71. 1999.
Ramos, O.H. and Selistre-de-Araujo, H.S., Biochem. Physiol. Toxicol. Pharmacol. 142,328-46. 2006.
Ribeiro, J.M. and Mather, T.N., Exp. Parasitol. 89, 213-21. 1998.
Ribeiro, J.M., et al. Insect Biochem. Mol. Biol. 36, 111-29. 2006.
Steen, N.A., et al. Toxicon 47, 1-20. 2005.
Valenzuela, J.G., Exp. Biol. 205, 2843-64. 2002.
Zouaoui Boudjeltia, K., B.M.C. Biotechnol. 2, 8. 2002.

SEQUENCE LISTING
(1) Information for SEQ ID N°:1
(i) Sequence characteritics:
(a) Length: 1467
(b) Type: nucleic acid
(ii) Molecule type: cDNA
(iii) Sequence description: SEQ ID N°1:
ATGTCGGGACTCAGCCTGAAATTGTGGATTGTAGCGTTCTTTTCTTTCTGCTTGGCCGA
GAAAGAGCATGGGATCGTGTACCCCAGGATGCTTGAAAGCAGAGCAGCAACTGGAGAGA
GAATGCTTAAAATCAACGATGACCTGACGTTGACGCTGCAGAAGAGTAAGGTCTTCGCT
GACGACTTTCTCTTCAGCACGACCGACGGAAATGAACCTATTGATTACTACATCAAAGC
CGAAGACGCTGAACGTGACATCTACCACGACGCAACTCACATGGCATCAGTAAGGGTAA
CGGACGATGATGGCGTGGAAGTGGAAGGAATTCTTGGAGAGAGGCTTCGTGTTAAACCT
TTGCCGGCAATGGCCCGCAGCAGCGATGGCCTCAGACCGCATATGTTGTACGAAGTCGA
CGCACACGAAAACGGCCGGCCACATGATTATGGTTCACCGAACACAACAAATACCCCCG
TAGAGAGAAGAGCTGGAGGCACAGAACCCCAGATGTACAAGATACCAGCGGAAATCTAT
CCCGAAGTTTACCTTGTGGCGGATAGTGCCTTTGCCAAAGAATTTAACTTTGATGTGAA
CGCCGTTACGCGTTACTTCGCAGTGCTTACAAATGCGGCTAATCTTAGGTATGAAAGCT
TCAAATCTCCAAAGGTACAGCTCAGGATCGTTGGCATAACGATGAACAAAAACCCAGCA
GACGAGCCATACATTCACAATATACGGGGATATGAGCAGTACCGGAATATTTTGTTTAA
GGAAACACTGGAGGATTTCAACACTCAGATGAAGTCAAAACATTTTTATCGTACTGCCG
ATATCGTGTTTCTCGTGACAGCAAAAAATATGTCCGAATGGGTTGGTAGCACACTACAA
TCATGGACTGGCGGGTACGCTTACGTAGGAACAGCGTGTTCCGAATGGAAAGTAGGAAT
GTGTGAAGACCGACCGACAAGCTATTACGGAGCTTACGTTTTCGCCCATGAGCTGGCGC
ATAATTTGGGTTGTCAACACGATGGAGATGGTGCCAATAGCTGGGTGAAAGGGCACATC
GGATCTGCGGACTGCCCATGGGATGACGGATACCTTATGAGCTACAAGATGGAAGACGA
GCGCCAGTATAAGTTTTCTCCCTGCTGCCAGAGAGAAGTCAGGAACCTCTACAGGCGTC
CGGAATTCAAATGCCTCACTGAACGAAAAGCGAAAAAAACAATCCGCTCGTCTAAGCTA
CCTGGTGTGATGACATCATCGAGCAACTATTGCCGGAGGGTGTACATGTACGAAAAAGG
CATGCACGCCGACGAGGCATATGGCGTCAAGGACTGCAGGGTAAAATGCACCACCACAT

CAAGAATGTATTGGCTACTCGGTGTAGTCGACGGTACACCTTGCGGAAATGGAAAGGCT
TGCATTCTTGGGAAATGCAGGAACAAAATCAAAATAAGCAAGAAGGACTGA

(2) Information for SEQ ID N°:2
(i) Sequence characteristics:
(a) Length: 1467
(b) Type: nucleic acid
(ii) Molecule type: cDNA
(iii) Sequence description: SEQ ID N°2:
   ATGTCGGAACTCAGCCTGAAATTGTGGACCGTAGCATTATTTTCTTTCTGCTTGGCCGA
   GAAAGAGCATGGGATCGTGTACCCCAGGATGCTTGAAAGCAGAGCTGCAACGGGAGAGA
   GGATGCTCAAAATCAACGATGACCTGACGTTGACGCTGCAGAAGAGTAAGGTCTTCGCT
   GACGACTTTCTCTTCAGCACGACAGAGGGAAATGAACCAATTGATTACTACATCAAAGC
   CGAAGATGCTGAGCGTGATATCTACCACGACTCAACTCACATGGCATCAGTAAGAGTAA
   CGGACGATGATGGCGTCGAAGTGGAAGGAATCCTTGGAGAGAGGCTTCGTGTTAAGCCT
   TTGCCGGCAATGGCCCGCAGCAGTGATGGCCTCAGACCGCATATGTTGTACGAAGTCGA
   CGCACACGAAAACGGCCGGCCACATGATTACGGCTCACCAAACACATCAAATACCCCCG
   TCGAATCAAGAGCTGGAAGCACCAAGCATAGTATGTACAAGATACCCATGGAAATCTTT
   CCCGAAGTTCACCTTGTGGTGGACAGCTTATTCGCCAAAGCTTTTAAATTCGACGTGTA
   CAAAGTGACCCGTTACTTTGCAGTGCTTACAAATGCGGCTAATCTTAGGTATGCCAGCT
   TCGTATTTCCAAAAGTACAGCTCAGGATCACTGGCATAACCATGAACAAAAAACCAAAC
   GACGAGCCATACATTCGCAATGTAAAGGGATATGAGAAGTACCGGAATATTTTGTTTAA
   GGAAACACTTGAGGATTTCAACACTTATATGAAATCAAAAAAATTCTATAATACCTCCG
   ATATTGTGTTCCTCGTGACAGCTAAAAATATGTCCGAATGGGTAGATGGCAAACTACAA
   CACTGGGTTGGAGGGTACGCTTATCTAGGAACAGCGTGTTCCCAATGGAAAGTAGGAAT
   GAGTGAAGACCCACCGACAAGCTATTACGGAGCCTACGTTTTCGCCCATGAGCTGGCGC
   ATAATCTGGGTTGTCAACACGACGGAGATGGTCCCAATGATTGGGTGAAAGGGCACATC
   GGATCTGCGGACTGCCCATGGGATGACGGATACCTCATGAGTTACAAGATGCAAGACGA
   GCGCCAGTATCAGTTCTCTTATTGCTGCCAGCGAGAAGTCAGGAACCTCTACAACCGTC
   CGGAATTCAAATGCCTCAGAGAACGATACACGACAAAAACAATAAAACGCTCGTCTAAG
   CTTCCCGGTGTGATGACAACACTGAGCAACTACTGCCAGAGGGTGTATAGGTACGACGA
   AGGCATGCACGCCGACAAGACATACGGCGTTCAAGACTGCAAGGTAAAATGCACCGGAA
   AACATATTTATTGGAGACTCAGTGTAGTCGACGGTACACCTTGCGGAAAGAAAAAGGCT
   TGCATTCTAGGAAAATGTGTGGATGCGATCAAAATAAGCAGAACGGACTAA

(3) Information for SEQ ID N°:3
(i) Sequence characteristics:
(a) Length: 1497
(b) Type: nucleic acid
(ii) Molecule type: cDNA
(iii) Sequence description: SEQ ID N°3:
ATGATGACTATCATTTTGCGGCTCCTTGGCTTCGCCGCAGTTGCACAGTGGAGCTCTGC
ATTTACCAACGAAGGGGCTCTTGTATACCCCCGACTTCTGGAAAGCCGGGGATCAAATG
GAGAGCGAATTCTGAAAATAAACGAGGATATTACCCTCAGCCTTGAGAGGACGTCAGTG
TTTCCTGAAAAACTGCTGATAAGGACACATGAAGAGGGATCGTTGGTCAATAATTATGT
CAATGGCTCCGAGCATAATGAACACCTATACCACGATACAGAAAAAATGGCGGCAGTCA
TTCTAAACGACGACGATGGAGTCAGTGTGGAAGGCCTGCTGCGCCACGACTTGCGAATC
CAGCCTATTCCACATTTGGAACGTTCATTGGAAGGTCACGTGGCCCATATGCTGTTCCC
TGTAAAGCAAAGAACGCCCTACCACGGAGATCACGGGCATCCAACCCACGAACTCAGTT
ATGCTCCTGAAGGCAATTCAACCTATACTGACTTTGCAGAGGAAAGAGGAGATCAAACC
ATATTGCCTGAAGTGCATGTGGTGCTTGATGCAACAACATGCGCTGCTTACAACTATAA
CAAGAAAGAAATTATCAACTATTTATCTGTAATGGCAATGTCGGCAAACCTGAGATATA
AAAGTGTCACACATCCATCCGTGAGATTGACCATCGTGGCTGTTACACTTCTTAAGGAA
AACACACAGCCTTTTATGAGATTTTACAAGGGAAACAAAGATATGGTCACATTTCAGGA
TACATTAATGGAATTTGACAACTATTACCAAAAGGATCCCGAGTTCAACAATGCCGACA
TTTATTTTCTACTCACAGGGCTTGACATAGTTGGGGTAGCGACTGATGGAACCTTGATG
CCACATTTCAGTGGGTACGCATATTTAAGCAAAGTCTGCACCGTATTCAAAGTAGGAAT
GGCCGAAGATGAACCGAAATCTTACGATGGTGTCCACCTCTTCAGCCACGAAATTGCCC
ATTTGCTAGGGTGCGCTCACGACGAGGACCCGCCGGATGGCACAATGCCAGGACATCCA
GGATCCCAAAACTGCCCCTGGAACGACGGTTACATCATGAGCTACGTCATAAACTTCAA
AAACCACTTCAAATTTTCTCCCTGCTGTGTCAGTTCAATTCGGTTCGTCGCAAAAGAGC
GGAAGTGCCTCTATGAGGTCAATGCCAAAAATCCAGTGAAAAACCTGAAATCTCTACCC
GGATTCAGGATATCGCCAACCAGTTTCTGTCAATTTATGCATCCACTTTACCGCGGTGT
CCATAGCGATAAGAAAGCAGGTTTGTCCGATTGCATACAGACGTGTAGAACGGCCAAAA
ATCGACGAGGAGGCTACAAGTCATGGACTCACGCGGCAATTGACGGGGTTCCTTGCGAC
AACAAAAACAGGAGGAAGGCCTGCATAAACGGGAGATGCACCCTTCTTGAGAGCATGCC
GGAGAGAACGTACCGGGAATGA

(4) Information for SEQ ID N°:4
(i) Sequence characteristics:
(a) Length: 1494
(b) Type: nucleic acid
ii) Molecule type: cDNA
(iii) Sequence description: SEQ ID N°4:
ATGACTACCATTTTGCGGCTCCTTGGCTTCGCCGCAGTGGCACAGTGGAGCTCTGCATT
TGCCGACGAAGGGGCGCTTGTATACCCCCGACTCCTAGAAGGCCGGGGAATAAATGGAG
AGAGAATCCTGAAAATAAATGAGGATATTACCCTCAACCTTGAAAGGACGTCTGTGTTC
CCTGAAAAACTGCTGATAAGGACACACGAAGATGGATCGTTGGTCAATACTTATGTCGA
CGGCTCGGACCATAACAAACACCTGTACCACGATACAAAACAAATGGCGGCAGTCATCC
TAAAAGACGAGGATGGAGTGAATGTGGAAGGCCTGCTGCGCCACGACTTGCGAATTCAA
CCTATGCCAGATTTGGAGCGGTCATTGGAAGGTCACGTGGCCCATATGCTGTTCCCTGT
GAAGCAAAGAACGCCCTACAGCGGAGATCACGGGCACCCACCAGATGAACTGAATAACG
TTCCTAAGGAGAATTCAACATATCCGGTTTTCCCAGAAGGAAGAGACAGTGGAACTATA
TCTCCGGAAGTACACGTGGTGCTTGATGCAACAACATGTGCAGCTTACAAATATGACAA
AAATGAAATTATCAACTATCTATCGGTAATGGCAACGGCGGCAAACCTAAGATATAGAA
GTGTTACTTATCCATCTGTAAGATTAACTATTGTGGCCGTTACACTTCTTCGGGAAAAT
ACGCAGCCATTTTTGAAATTTTACCAGCAAAGCAAAGATATGATCACATTTCATAATAC
ATTAATGGAATTCGGCTCGCATTACCAAAAGGATCCCGAATTCAATAATGCCGATATTT
ATTTTCTGCTCACAGGGCTTGATATAGTTGGGGTAGCGACGGACGGAACCTTAATGCCA
CAATTCAGCGGGTATGCCTATTTAAGCAAAGTCTGCACCGTATTCAAAGTAGGAATGTC
AGAAGATGAACCCAAGTCCTACGACGGTGTCCACCTCTTCAGCCACGAAATCGCCCACT
TGCTAGGGTGCGCTCACGACGAGGACCCCCCGGATGGCACAATGCCAGGACATCCGGGA
TCCCAGAAGTGCCCCTGGAACGACGGTTACATCATGAGCTACGTCATAAACTTCAAAAA
CCACTTCAAATTTTCTCCCTGCTGTGTAGAATCAATTCGATTCGTCGCACGAGAGCGGG
ACTGCCTCTACAAAGTCAATGCCAAGGATGCTGTAAAAAGCCTAATATCTCTGCCCGGA
TTTAGGATATCGCCAACGAGTTTCTGTCAATTTATGCATCCGCTTTACCGCGGTGTCCA
TAGCGATAAGAAAGCAGGTCTGTCCGATTGCGTACAGACGTGTAGAACGGCCAAAAATC
GACGAGGAGGCTACCATTCATGGATTCACGCGGCACTTGACGGGGTTCCTTGCGACAAG
AGAAACCCCAAGAAGGCCTGCATAAACGGGAAATGCACCCTCCTTAAGAGCATGCCCCA
GAGAACGTACCGGGAATGA

(5) Information for SEQ ID N°:5
(i) Sequence characteristics:
(a) Length: 1584
(b) Type: nucleic acid
(ii) Molecule type: cDNA
(iii) Sequence description: SEQ ID N°5:
ATGTTGTGGCTCTATGTGTTGGTGGTTGTTTTGTCGGCGTCGTTGGACCTCACTCTTTC
AGGGCCTACGGCGGAGCGCGTTGTTTATCCACGGCTGCTACAAGCAAGAGGAGCGAATG
GTGAGAAGTTGCTTCACATTCGAAATGGACTGACGCTTCACTTGGAAAAGACCTCTGTC
TTGGCTGAGAACTTCATCCTCACCACATTCGAAGGCGGAAATCAAATTCATACCGCGAT
GAACGGGAAAGACCTGGAAAAAAACTTGTACCGAGACCGAAACCAGGCGGCTGCAATAT
CAGTCGAAGAACAGGATGGAACGTTAGAAGTGAGAGGAGCACTGAGCCCCAAGCTGAGG
ATTGCTCCTTCGCCACTCAAGGCTCGATCCGAAGATGGGCAGATTGCGCATGAAATATT
CGAAATCGAGCAGAATGGTGAATTTAGGAGCGATTCTATCGTGCCACCCAGTTTGAAAG
TGCAAGAACGTACAGTGGTTTACCGTAACAAGTACACCAGAGTTCCTGTAAATTTTACC
GTCGAAGTTGCCATGCTGATTGATACGTATTTATACAAGGAGTTCAAGAACGAGAGCCA
CATCGTACCGTACCTGGCTATGATACTGACTTTGATAAATATGAGGTATGATGACACAC
ATGACCCGTACATCCAGTTTCTTCTGACGCAAGTGTTCGTGGGGAAAACTGGCGATCCA
GTTTCAGAGACTATGTACGAGTATGACGTAAAAATGCCAAGCGGACCCAAGAAGTTATA
CATGCAGTCGGAGATAACTCTGGCCAGCCTAGCAAAGGCCGTGAAGTATCGGGTGCTCG
ACACTACTGCGGATATAATGATCCTGGTGACTGGACTTGATTTGGCAGATAAGGAGGGC
GGCAAAGTAGACAATTCCGTCTTGGGAATTGCGTACCTTGGTGCCGTATGCAGCGTAGG
TTTACGAGCTGCTCTTGCTGAGGACCACGCCTACACGTTTTCCACGGTTGGCGTGACTG
CGCACGAACTGGCTCACGCTCTTGGAAGTGTTCACGACGGAGATCAACCCATCTATGCC
ACCGTCGGAAAACGAGTCAGCGGCTGTAACGCACATGATGAATATACTATGGCGCCTGT
CGCTGGCGGCAAGAATTTCGGAAAGTTCTCCATCTGTTCCCTGAATCAACTGTCGTCCT
TCGCCGGGACATTATCCCAAAGATGCTTTAACATTTCGGAGTCGAGTACCTTCAAAATG
CCCACAAAACCAATTCCGGGCACAAAGTGGAACCCGTTTCCGGGACGAGCCTTAGACAA
GACATTCTACTGCAAGAGTTTATACGCGAAATCATGGCGTGTAACAGCACGTGACCACC
TCGACTACGCGCGCCGATGCAAGCTACTATGCTGCCCCTCAACTTACGGAAGGTGCTAC
GTTCACGACATGGTGGATGGAATGATATGTGGCTATCAAAAGGTGTGTATGAGACACGT
GTGCGCAAGACCAGGCGAGCATCCAGCGTCACCACCCCGGCGGGCGACCACAGCAACCC
CGACGCGTAATAATGGACGTTATAATTACTGGGGACGTCGAAGTGGCTAA

Amino acid sequences.

(1) Information for SEQ ID N°:6
(i) Sequence characteristics:
(a) Length: 488
(b) Type: amino acid
(ii) Molecule type: polypeptide
(iii) Sequence description: SEQ ID N°6:
MSGLSLKLWIVAFFSFCLAEKEHGIVYPRMLESRAATGERMLKINDDLTLTLQKSKVFA
DDFLFSTTDGNEPIDYYIKAEDAERDIYHDATHMASVRVTDDDGVEVEGILGERLRVKP
LPAMARSSDGLRPHMLYEVDAHENGRPHDYGSPNTTNTPVERRAGGTEPQMYKIPAEIY
PEVYLVADSAFAKEFNFDVNAVTRYFAVLTNAANLRYESFKSPKVQLRIVGITMNKNPA
DEPYIHNIRGYEQYRNILFKETLEDFNTQMKSKHFYRTADIVFLVTAKNMSEWVGSTLQ
SWTGGYAYVGTACSEWKVGMCEDRPTSYYGAYVFAHELAHNLGCQHDGDGANSWVKGHI
GSADCPWDDGYLMSYKMEDERQYKFSPCCQREVRNLYRRPEFKCLTERKAKKTIRSSKL
PGVMTSSSNYCRRVYMYEKGMHADEAYGVKDCRVKCTTTSRMYWLLGVVDGTPCGNGKA
CILGKCRNKIKISKKD

(2) Information for SEQ ID N°:7
(i) Sequence characteristics:
(a) Length: 488
(b) Type: amino acid
(ii) Molecule type: polypeptide
(iii) Sequence description: SEQ ID N°7:
MSELSLKLWTVALFSFCLAEKEHGIVYPRMLESRAATGERMLKINDDLTLTLQKSKVFA
DDFLFSTTEGNEPIDYYIKAEDAERDIYHDSTHMASVRVTDDDGVEVEGILGERLRVKP
LPAMARSSDGLRPHMLYEVDAHENGRPHDYGSPNTSNTPVESRAGSTKHSMYKIPMEIF
PEVHLVVDSLFAKAFKFDVYKVTRYFAVLTNAANLRYASFVFPKVQLRITGITMNKKPN
DEPYIRNVKGYEKYRNILFKETLEDFNTYMKSKKFYNTSDIVFLVTAKNMSEWVDGKLQ
HWVGGYAYLGTACSQWKVGMSEDPPTSYYGAYVFAHELAHNLGCQHDGDGPNDWVKGHI
GSADCPWDDGYLMSYKMQDERQYQFSYCCQREVRNLYNRPEFKCLRERYTTKTIKRSSK
LPGVMTTLSNYCQRVYRYDEGMHADKTYGVQDCKVKCTGKHIYWRLSVVDGTPCGKKKA
CILGKCVDAIKISRTD

(3) Information for SEQ ID N°:8
(i) Sequence characteristics:
(a) Length: 498
(b) Type: amino acid
(ii) Molecule type: polypeptide
(iii) Sequence description: SEQ ID N°8:
MMTIILRLLGFAAVAQWSSAFTNEGALVYPRLLESRGSNGERILKINEDITLSLERTSV
FPEKLLIRTHEEGSLVNNYVNGSEHNEHLYHDTEKMAAVILNDDDGVSVEGLLRHDLRI
QPIPHLERSLEGHVAHMLFPVKQRTPYHGDHGHPTHELSYAPEGNSTYTDFAEERGDQT
ILPEVHVVLDATTCAAYNYNKKEIINYLSVMAMSANLRYKSVTHPSVRLTIVAVTLLKE
NTQPFMRFYKGNKDMVTFQDTLMEFDNYYQKDPEFNNADIYFLLTGLDIVGVATDGTLM
PHFSGYAYLSKVCTVFKVGMAEDEPKSYDGVHLFSHEIAHLLGCAHDEDPPDGTMPGHP
GSQNCPWNDGYIMSYVINFKNHFKFSPCCVSSIRFVAKERKCLYEVNAKNPVKNLKSLP
GFRISPTSFCQFMHPLYRGVHSDKKAGLSDCIQTCRTAKNRRGGYKSWTHAAIDGVPCD
NKNRRKACINGRCTLLESMPERTYRE

(4) Information for SEQ ID N°:9
(i) Sequence characteristics:
(a) Length: 497
(b) Type: amino acid
(ii) Molecule type: polypeptide
(iii) Sequence description: SEQ ID N°9:
MTTILRLLGFAAVAQWSSAFADEGALVYPRLLEGRGINGERILKINEDITLNLERTSVF
PEKLLIRTHEDGSLVNTYVDGSDHNKHLYHDTKQMAAVILKDEDGVNVEGLLRHDLRIQ
PMPDLERSLEGHVAHMLFPVKQRTPYSGDHGHPPDELNNVPKENSTYPVFPEGRDSGTI
SPEVHVVLDATTCAAYKYDKNEIINYLSVMATAANLRYRSVTYPSVRLTIVAVTLLREN
TQPFLKFYQQSKDMITFHNTLMEFGSHYQKDPEFNNADIYFLLTGLDIVGVATDGTLMP
QFSGYAYLSKVCTVFKVGMSEDEPKSYDGVHLFSHEIAHLLGCAHDEDPPDGTMPGHPG
SQKCPWNDGYIMSYVINFKNHFKFSPCCVESIRFVARERDCLYKVNAKDAVKSLISLPG
FRISPTSFCQFMHPLYRGVHSDKKAGLSDCVQTCRTAKNRRGGYHSWIHAALDGVPCDK
RNPKKACINGKCTLLKSMPQRTYRE

(5) Information for SEQ ID N°:10
(i) Sequence characteristics:
(a) Length: 527
(b) Type: amino acid
(ii) Molecule type: polypeptide
(iii) Sequence description: SEQ ID N°10:
MLWLYVLVVVLSASLDLTLSGPTAERVVYPRLLQARGANGEKLLHIRNGLTLHLEKTSV
LAENFILTTFEGGNQIHTAMNGKDLEKNLYRDRNQAAAISVEEQDGTLEVRGALSPKLR
IAPSPLKARSEDGQIAHEIFEIEQNGEFRSDSIVPPSLKVQERTVVYRNKYTRVPVNFT
VEVAMLIDTYLYKEFKNESHIVPYLAMILTLINMRYDDTHDPYIQFLLTQVFVGKTGDP
VSETMYEYDVKMPSGPKKLYMQSEITLASLAKAVKYRVLDTTADIMILVTGLDLADKEG
GKVDNSVLGIAYLGAVCSVGLRAALAEDHAYTFSTVGVTAHELAHALGSVHDGDQPIYA
TVGKRVSGCNAHDEYTMAPVAGGKNFGKFSICSLNQLSSFAGTLSQRCFNISESSTFKM
PTKPIPGTKWNPFPGRALDKTFYCKSLYAKSWRVTARDHLDYARRCKLLCCPSTYGRCY
VHDMVDGMICGYQKVCMRHVCARPGEHPASPPRRATTATPTRNNGRYNYWGRRSG

## Claims

1. An isolated and purified polypeptide obtained from tick salivary gland and presenting more than 75% sequence identity with at least a sequence selected from the group consisting of SEQ.ID.NO.8 or SEQ.ID.NO.9.

2. The polypeptide of claim 1, which presents at least 80% sequence identity with SEQ.ID.NO.8 or SEQ.ID.NO.9.

3. The polypeptide according to claim 1 or 2, which presents at least 90%, sequence identity with SEQ.ID.NO.8 or SEQ.ID.NO.9.

4. The polypeptide according to any of the preceding claims 1 to 3, which presents at least 95% sequence identity with SEQ.ID.NO.8 or SEQ.ID.NO.9.

5. The polypeptide according to any of the preceding claims 1 to 4, which presents at least 98 to 99% sequence identity with SEQ.ID.NO.8 or SEQ.ID.NO.9.

6. A polypeptide comprising or consisting of the sequence SEQ.ID.NO.8, SEQ.ID.NO.9 or a biologically active fragment thereof.

7. The polypeptide according to claims 1 to 6 modified by or linked to at least one substitution group, preferably selected from the group consisting of amide, acetyl, phosphoryl, and/or glycosyl groups.

8. The polypeptide according to claims 1 to 7 in the form of a "mature" protein.

9. The polypeptide according to claims 1 to 7 as part of a larger protein.

10. The polypeptide according to claims 1 to 7 as part of a fusion protein.

11. The polypeptide according to any of the preceding claims 1 to 10 further including at least one additional amino acid sequence which contains secretory or leader sequences, pro-sequences, sequences which help in purification such as multiple histidine residues, or additional sequences for stability during recombination protection.

12. A polynucleotide sequence encoding the polypeptide according to any of the preceding claims 1 to 11, preferably having the sequence SEQ.ID.NO.3 or SEQ.ID.NO.4.

13. A vector comprising at least one element selected from the group consisting of the polypeptide according to any of the claims 1 to 11 or the polynucleotide of claim 12.

14. A cell transfected or comprising the vector according to claim 13.

15. An (monoclonal or polyclonal) antibody or an hypervariable fragment thereof raised against the polynucleotide according to any of the claims 1 to 7 or the polypeptide according to any of the claims 8 to 13.

16. A hybridoma cell line expressing the antibody according to claim 15.

17. A pharmaceutical composition comprising an adequate pharmaceutical carrier and an element selected from the group consisting of the polypeptide according to any of the claims 1 to 11, the polynucleotide according to the claim 12, the vector according to claim 11, the cell according to claim 14, the antibody according to claim 15, or a mixture thereof.

18. An immunological composition or vaccine for inducing an immunological response in a mammalian host to a tick salivary gland polypeptide which comprises at least one element of the group consisting of:
a) the tick salivary gland polypeptide according to any of the claims 1 to 11;
b) the tick salivary gland polynucleotide according to any of the claim 12;
c) epitope-bearing fragments, analogs, outer-membrane vesicles or cells (attenuated or otherwise) of components a) or b);
d) possibly an adequate pharmaceutical carrier.

19. Use of the immunological composition or vaccine according to claim 18 or the vector according to claim 13 for the manufacture of a medicament to produce antibody and/or T-cell immune response to protect a mammal from bacteria and viruses and related species and in the treatment or the prevention of Lyme diseases or tick encephalitis virus diseases.

20. Use of the pharmaceutical composition according to claim 17 for the manufacture of a medicament in the treatment or the prevention of a disease selected from the group consisting of cardiovascular disease, cancer, inflammation, graft rejection, auto immune diseases or allergy.

21. A diagnostic kit for detecting a disease or susceptibility to a disease induced or transmitted by tick, especially *Ixodes ricinus*, which comprises:
a) the tick salivary gland polypeptide according to the claims 1 to 11;
b) the tick salivary gland polynucleotide according to the claim 12;
c) the antibody according to claim 15;
d) a phage displaying the antibody according to claim 15.
